# EUROPEAN PATENT APPLICATION

(11) **EP 4 843 977 A1**
(43) Date of publication of application: **25.11.2026**
(21) Application number: 24918954.9
(22) Date of filing: 12.11.2024
(51) Int. Cl.: C07C 63/26, C07C 51/09, C08G 63/183, C08G 63/199, C08G 63/672

(54) **RECYCLED TEREPHTHALIC ACID AND POLYESTER RESIN COMPRISING SAME**

(30) Priority: 17.01.2024 KR 20240007491
(71) Applicant: SK Chemicals Co., Ltd., Seongnam-si, Gyeonggi-do 13494 (KR)
(72) Inventor: HWANG, Da-Young, Seongnam-si, Gyeonggi-do 13494 (KR); KANG, Ju-Sik, Seongnam-si, Gyeonggi-do 13494 (KR); LEE, Yoo Jin, Seongnam-si, Gyeonggi-do 13494 (KR); CHAE, Hee-Il, Seongnam-si, Gyeonggi-do 13494 (KR)
(74) Representative: Dehns
(86) International application number: PCT/KR2024/017802
(87) International publication number: WO 2025/154920

(57) **Abstract**

The present invention relates to a recycled terephthalic acid produced from waste polyester and a polyester resin comprising same. Specifically, according to an embodiment of the present invention, the recycled terephthalic acid is obtained by alcoholysis and hydrolysis of waste polyester, and color b measured in a reflection mode of a spectrophotometer satisfies less than 2, and thus the recycled terephthalic acid is environmentally friendly, has a low pigment residual rate and a low yellow index, and also has a very low content of metal, which is an impurity. Therefore, the polyester resin comprising the recycled terephthalic acid has excellent quality without further comprising a terephthalic acid in addition to the recycled terephthalic acid.

## Description

### Technical Field

The present invention relates to recycled terephthalic acid prepared from waste polyester and to a polyester resin comprising the same.

### Background Art

Polyester is widely used as a material for beverage-filling containers, packaging films, audio and video films, and the like, or an industrial material such as medical fibers and tire cords, by virtue of its excellent mechanical strength, thermal resistance, transparency, and gas barrier properties. In particular, polyester sheets or plates have good transparency and excellent mechanical strength, so that they are widely used as raw materials for cases, boxes, partitions, shelves, panels, packaging materials, building materials, interior and exterior materials, and the like.

As waste of plastics such as polyester is generated globally at an unmanageable level every year, there is increasing interest in recycling waste polyester or regeneration processes using waste polyester. In addition, countries around the world have prepared regulations and plans for recycling waste plastic resources, including waste polyester. For example, regulations that require a recycled resin to be used at a certain ratio or more in packaging materials used in various fields are being discussed.

In particular, since polyethylene terephthalate (PET) has excellent properties in terms of thermal resistance, processability, transparency, and non-toxicity, it is widely used to manufacture a wide range of products such as films, fibers, bottles, and containers. However, most of them after use are landfilled or incinerated; thus, research on recycling or regeneration processes using it is continuing.

For example, Korean Laid-open Patent Publication No. 1997-0042469 discloses a technology for preparing terephthalic acid by hydrolyzing waste polyethylene terephthalate with an aqueous alkaline solution to obtain a slurry of alkali metal and earth metal salts of terephthalic acid and neutralizing it with an acid. A terephthalic acid salt, rather than terephthalic acid, is produced as a result of the hydrolysis reaction, and a neutralization step by adding an acid is necessary to convert it to terephthalic acid. There is a problem in that environmental pollutants are generated from the by-products formed as a result, or a large amount of acid treatment waste solutions is generated. In addition, as described above, in the case of terephthalic acid converted from a terephthalic acid salt, the purity is low since impurities are not readily removed, and the physical properties such as color characteristics are poor, resulting in poor quality.

### [Prior Art Document]

### [Patent Document]

(Patent Document 1) Korean Laid-open Patent Publication No. 1997-0042469

### Detailed Description of the Invention

### Technical Problem

Accordingly, the present invention aims to provide terephthalic acid with excellent quality that is produced in an environmentally friendly manner by alcoholysis and hydrolysis of waste polyester, and a polyester resin comprising the same.

### Solution to the Problem

The recycled terephthalic acid according to an embodiment of the present invention is obtained by alcoholysis and hydrolysis of waste polyester, and it has a Color b of less than 2 as measured in the reflection mode of a spectrophotometer.

The polyester resin according to another embodiment of the present invention comprises recycled terephthalic acid obtained by alcoholysis and hydrolysis of waste polyester and has a Color b of less than 2 as measured in the reflection mode of a spectrophotometer, and it has a difference between the Color L value and the Color b value (Color L - Color b) of greater than 83 as measured in the transmission mode of a spectrophotometer for a specimen thereof having a thickness of 6 mm.

The article according to another embodiment of the present invention comprises the polyester resin.

The process for preparing a polyester resin according to another embodiment of the present invention comprises conducting alcoholysis and hydrolysis of waste polyester to prepare recycled terephthalic acid; mixing the recycled terephthalic acid with a glycol component to prepare a preliminary composition; subjecting the preliminary composition to an esterification reaction; and subjecting the esterification reaction product to a polycondensation reaction, wherein the recycled terephthalic acid has a Color b of less than 2.

### Advantageous Effects of the Invention

As the recycled terephthalic acid according to an embodiment of the present invention is obtained by alcoholysis and hydrolysis of waste polyester, and it has a Color b of less than 2 as measured in the reflection mode of a spectrophotometer, it is environmentally friendly, has a low pigment residual rate and a low yellow index, and has a very low content of metal impurities, resulting in excellent quality.

Therefore, a polyester resin comprising the recycled terephthalic acid has excellent quality even without additionally comprising terephthalic acid other than the recycled terephthalic acid. Specifically, the physical properties such as color characteristics of conventional recycled terephthalic acid prepared from waste polyester are not good. In addition, the quality of a polyester resin prepared using such recycled terephthalic acid alone as an acid component is low as well, making it difficult to be used. Therefore, in order to secure the properties of a polyester resin, the content of recycled terephthalic acid is reduced and used in small quantities, or commercially available terephthalic acid is used together.

However, the polyester resin according to an embodiment of the present invention comprises recycled terephthalic acid having excellent quality although it is prepared from waste polyester and has a low pigment residual rate and a low yellow index, as well as a very low content of metal impurities. As a result, it has excellent quality even when it comprises the recycled terephthalic acid alone as an acid component or comprises the recycled terephthalic acid at a higher content than the prior art.

### Best Mode for Carrying out the Invention

Hereinafter, the present invention will be described in detail. The present invention is not limited to the disclosures given below, but it may be modified into various forms as long as the gist of the invention is not changed.

Throughout the present specification, when a part is referred to as "comprising" an element, it is understood that other elements may be comprised, rather than other elements are excluded, unless specifically stated otherwise.

All numbers and expressions related to the quantities of components, reaction conditions, and the like used herein are to be understood as being modified by the term "about," unless otherwise indicated.

Throughout the present specification, the terms first, second, and the like are used to describe various components. But the components should not be limited by the terms. The terms are used only for the purpose of distinguishing one component from another.

### Recycled terephthalic acid

The recycled terephthalic acid according to an embodiment of the present invention is obtained by alcoholysis and hydrolysis of waste polyester, and it has a Color b of less than 2 as measured in the reflection mode of a spectrophotometer.

The alcoholysis and hydrolysis will be described in detail below.

The recycled terephthalic acid according to an embodiment of the present invention has a Color b of less than 2 as measured in the reflection mode of a spectrophotometer. For example, the Color b of the recycled terephthalic acid may be 1.9 or less, 1.8 or less, 1.6 or less, 1.4 or less, 1.2 or less, or 1 or less, as measured in the reflection mode of a spectrophotometer. Since the above numerical range of Color b is equivalent to that of terephthalic acid commonly produced in the petrochemical processes, the recycled terephthalic acid that satisfies the above range of Color b is not only low in yellow index but also excellent in quality as the monomer is well purified.

Color b is a color coordinate established by the Commission International d'Eclairage (CIE), where color is represented by L (brightness), a (green to red complementary color), and b (yellow to blue complementary color). It can be measured using a spectrophotometer.

More specifically, the recycled terephthalic acid is measured in the reflectance mode with Illuminant D65 at an observer angle of 2° using a Varian Cary 5 UV/Vis/NIR spectrophotometer equipped with a diffuse reflectance accessory capable of measuring color and brightness to obtain data, and the Color b value is calculated by processing it with the color analysis device in the Grams/32 software.

The recycled terephthalic acid is obtained through a purification process and may have a pigment residual rate of 10% or less according to the following Equation 1. [Equation 1] $Pigment residual rate of recycled terephthalic acid \left(%\right) = \frac{B 1}{B 2} \times 100$

In Equation 1, B1 is the area of an absorbance curve obtained in 400 nm to 800 nm using a UV-vis spectrophotometer for the recycled terephthalic acid as diluted to a concentration of 5% in dimethyl sulfoxide (DMSO), dimethyl formamide (DMF), or methylpyrrolidone (NMP), and B2 is the area of an absorbance curve obtained in the same manner as above for recycled terephthalic acid that is obtained by the same preparation process as above but is not subjected to the purification process.

B1 may be measured for recycled terephthalic acid produced by performing a purification step, or a purification and concentration step, in the process for preparing recycled terephthalic acid. B2 may be measured for recycled terephthalic acid produced without performing a purification step, or by performing a concentration step alone, in the process for preparing recycled terephthalic acid.

The pigment residual rate refers to the content of additives such as colorants, pigments, dyes, or the like remaining in a composition. The lower the pigment residual rate, the lower the content of additives such as colorants, pigments, dyes, or the like, indicating higher purity.

For example, the pigment residual rate of the recycled terephthalic acid may be 9% or less, 8% or less, 7% or less, 5% or less, or 4% or less.

As a specific example, when dimethyl sulfoxide (DMSO) is used as a solvent for diluting the recycled terephthalic acid, the pigment residual rate according to the above Equation 1 may be 2% or less, 1% or less, or 0.5% or less. When dimethylformamide (DMF) is used, the pigment residual according to the above Equation 1 may be 2% or less, 1% or less, or 0.5% or less. When methylpyrrolidone (NMP) is used, the pigment residual according to the above Equation 1 may be 2% or less, 1% or less, or 0.5% or less.

The purification and concentration processes will be described in detail below.

In addition, the yellow index (Y.I.) may be less than 2, 1.9 or less, 1.8 or less, or 1.7 or less, when the recycled terephthalic acid is diluted to a concentration of 5% in dimethyl sulfoxide (DMSO), dimethyl formamide (DMF), or methylpyrrolidone (NMP) and measured with a spectrophotometer.

As a specific example, the yellow index may be 2 or less, 1.8 or less, or 1.6 or less, when the recycled terephthalic acid is diluted to a concentration of 5% in dimethyl sulfoxide (DMSO) and measured with a spectrophotometer. The yellow index may be 2 or less, 1.9 or less, or 1.6 or less, when the recycled terephthalic acid is diluted to a concentration of 5% in dimethyl formamide (DMF) and measured with a spectrophotometer. The yellow index may be 2 or less, 1.7 or less, or 1.6 or less, when the recycled terephthalic acid is diluted to a concentration of 5% in methylpyrrolidone (NMP) and measured with a spectrophotometer.

The yellow index may be measured for recycled terephthalic acid produced by performing a purification step, or a purification and concentration step, in the process for preparing recycled terephthalic acid.

According to an embodiment of the present invention, the recycled terephthalic acid may have a total content of metals of less than 100 ppm when measured by inductively coupled plasma atomic emission spectroscopy (ICP-AES). Specifically, the recycled terephthalic acid, more specifically, the recycled terephthalic acid prepared by carrying out a purification step, or purification and concentration steps, may have a low content of insoluble impurities such as metals.

For example, the recycled terephthalic acid may have a total content of metals of 90 ppm or less, 80 ppm or less, 65 ppm or less, 50 ppm or less, 35 ppm or less, less than 30 ppm, 15 ppm or less, 9 ppm or less, 7 ppm or less, 5 ppm or less, or 1 ppm or less, when measured by inductively coupled plasma atomic emission spectroscopy (ICP-AES).

In addition, the recycled terephthalic acid may have a total content of Sb, Ti, and Zn of less than 30 ppm when measured by inductively coupled plasma atomic emission spectroscopy (ICP-AES). For example, the total content of Sb, Ti, and Zn, which may be harmful to the human body or have the potential to serve as a catalyst for reaction or side reaction in the subsequent polymerization process, in the recycled terephthalic acid may be 25 ppm or less, 20 ppm or less, 15 ppm or less, 10 ppm or less, 5 ppm or less, 3 ppm or less, or 1 ppm or less.

For example, the content of Sb in the recycled terephthalic acid may be 30 ppm or less, 25 ppm or less, 20 ppm or less, 15 ppm or less, 10 ppm or less, 5 ppm or less, 3 ppm or less, or 1 ppm or less, relative to the total weight of the recycled terephthalic acid, when measured by inductively coupled plasma atomic emission spectroscopy (ICP-AES).

The content of Ti in the recycled terephthalic acid may be 30 ppm or less, 25 ppm or less, 20 ppm or less, 15 ppm or less, 10 ppm or less, 5 ppm or less, 3 ppm or less, or 1 ppm or less, relative to the total weight of the recycled terephthalic acid, when measured by inductively coupled plasma atomic emission spectroscopy (ICP-AES).

The content of Zn in the recycled terephthalic acid may be 30 ppm or less, 25 ppm or less, 20 ppm or less, 15 ppm or less, 10 ppm or less, 5 ppm or less, 3 ppm or less, or 1 ppm or less, relative to the total weight of the recycled terephthalic acid, when measured by inductively coupled plasma atomic emission spectroscopy (ICP-AES).

### Polyester resin

The polyester resin according to another embodiment of the present invention comprises recycled terephthalic acid obtained by alcoholysis and hydrolysis of waste polyester and has a Color b of less than 2 as measured in the reflection mode of a spectrophotometer, and it has a difference between the Color L value and the Color b value (Color L - Color b) of greater than 83 as measured in the transmission mode of a spectrophotometer for a specimen thereof having a thickness of 6 mm.

The polyester resin comprises the recycled terephthalic acid.

Specifically, the content of recycled terephthalic acid may be 0.1% by weight to 55% by weight based on the total weight of the polyester resin. For example, the content of recycled terephthalic acid may be 0.5% by weight to 55% by weight, 1% by weight to 52% by weight, 1.5% by weight to 50% by weight, 2% by weight to 48% by weight, 3% by weight to 46% by weight, 3.5% by weight to 45% by weight, or 10% by weight to 50% by weight, based on the total weight of the polyester resin.

In addition, the polyester resin has a difference between the Color L value and the Color b value (Color L - Color b) of greater than 83, 84 or more, 85 or more, 88 or more, 89 or more, or 90 or more, as measured in the transmission mode of a spectrophotometer for a specimen thereof having a thickness of 6 mm.

More specifically, the polyester resin is prepared into a specimen having a thickness of 6 mm. This specimen is measured in the transmission mode with Illuminant D65 at an observer angle of 2° using a Varian Cary 5 UV/Vis/NIR spectrophotometer equipped with a diffuse reflectance accessory capable of measuring color and brightness to obtain data, and the Hunter Color L, Color a, and Color b values are calculated by processing it with the color analysis device in the Grams/32 software. Then, the Color L - Color b value, which is the difference between the Color L value and the Color b value, is calculated.

In addition, the polyester resin may have an intrinsic viscosity (IV) of 0.5 dl/g to 1.3 dl/g. For example, the intrinsic viscosity (IV) of the polyester resin may be 0.5 dl/g to 1.2 dl/g, 0.55 dl/g to 1 dl/g, or 0.6 dl/g to 0.95 dl/g.

As an embodiment of the present invention, the polyester resin may comprise a diacid component and a glycol component. Specifically, the polyester resin may comprise the recycled terephthalic acid alone as a diacid component, or it may comprise another diacid component together with the recycled terephthalic acid. More specifically, the polyester resin is a resin in which a diacid component and a diol component are copolymerized, and it may comprise a repeat unit derived from the diacid component and a repeat unit derived from the diol component.

Conventionally, recycled terephthalic acid prepared from waste polyester has poor physical properties such as color characteristics, making it difficult to be used alone as an acid component as a raw material for a polyester resin. However, the polyester resin according to an embodiment of the present invention is prepared using the recycled terephthalic acid; thus, sufficient physical properties can be secured even when the content of the recycled terephthalic acid is increased relative to the conventional one. Therefore, the polyester resin according to an embodiment of the present invention may comprise the recycled terephthalic acid alone or may comprise it together with another diacid component to enhance physical properties and the like.

The diacid component may comprise at least one selected from the group consisting of terephthalic acid (TPA), isophthalic acid (IPA), dimethyl isophthalate, bis(2-hydroxyethyl) terephthalate, recycled bis(2-hydroxyethyl) terephthalate (r-BHET), phthalic acid, dimethyl terephthalate, phthalic anhydride, 2,6-naphthalenedicarboxylic acid, dimethyl 2,6-naphthalenedicarboxylate, diphenyl dicarboxylic acid, 1,4-cyclohexanedicarboxylic acid, 1,3-cyclohexanedicarboxylic acid, dimethyl 1,4-cyclohexanedicarboxylate, dimethyl 1,3-cyclohexanedicarboxylate, sebacic acid, succinic acid, isodecylsuccinic acid, maleic acid, maleic anhydride, fumaric acid, adipic acid, glutaric acid, and azelaic acid.

In addition, the glycol compound may comprise at least one selected from the group consisting of isosorbide (ISB), recycled isosorbide (r-ISB), ethylene glycol (EG), recycled ethylene glycol (r-EG), 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, 1,4-cyclohexanediol, cyclohexanedimethanol (CHDM) and its derivatives, recycled cyclohexanedimethanol (r-CHDM), diethylene glycol (DEG), recycled diethylene glycol (r-DEG), neopentyl glycol (NPG), and recycled neopentyl glycol (r-NPG).

Specifically, the polyester resin may comprise the recycled terephthalic acid and the diacid component. In such an event, the molar ratio of the recycled terephthalic acid and the diacid component may be 1:0.01 to 3.5, 1:0.02 to 3.3, 1:0.03 to 3.0, or 1:0.05 to 2.6.

According to an embodiment of the present invention, the polyester resin may further comprise at least one additive selected from the group consisting of a colorant, a crystallizing agent, an oxidation stabilizer, and a branching agent.

The colorant is an additive for enhancing the color characteristics of the polyester resin. Commonly used colorants such as cobalt acetate and cobalt propionate may be used as the colorant as long as the effects of the present invention are not impaired.

Specifically, the colorant may be cobalt acetate, cobalt propionate, anthraquinone-based compounds, perinone-based compounds, azo-based compounds, and methine-based compounds.

In addition, the polyester resin may comprise the colorant in an amount of 0.1 ppm to 30 ppm based on the total weight of the polyester resin. For example, the colorant may be employed in an amount of 0.2 ppm to 30 ppm, 0.5 ppm to 25 ppm, 0.6 ppm to 23 ppm, or 0.8 ppm to 20 ppm, based on the total weight of the polyester resin. As the content of the colorant satisfies the above range, the color characteristics of the polyester resin may be sufficiently enhanced without deteriorating its mechanical properties.

The crystallizing agent may comprise at least one selected from the group consisting of a crystal nucleating agent, a UV absorber, a polyolefin resin, a polyamide resin, and a polyalkylene resin.

In addition, the polyester resin may comprise the crystallizing agent in an amount of 0.1 ppm to 10 ppm based on the total weight of the polyester resin. For example, the crystallizing agent may be employed in an amount of 0.2 ppm to 8 ppm, 0.5 ppm to 6 ppm, 1 ppm to 10 ppm, 2 ppm to 8 ppm, 3 ppm to 6 ppm, or 4 ppm to 6 ppm, based on the total weight of the polyester resin. As the content of the crystallizing agent satisfies the above range, thermal resistance and impact strength can be enhanced.

The oxidation stabilizer may comprise at least one selected from the group consisting of hindered phenol-based oxidation stabilizers, phosphite-based oxidation stabilizers, and thioether-based oxidation stabilizers.

In addition, the polyester resin may comprise the oxidation stabilizer in an amount of 50 ppm to 2,500 ppm based on the total weight of the polyester resin. For example, the oxidation stabilizer may be employed in an amount of 50 ppm to 2,300 ppm, 60 ppm to 2,200 ppm, 80 ppm to 2,100 ppm, 100 ppm to 2,000 ppm, or 100 ppm to 1,500 ppm, based on the total weight of the polyester resin. As the content of the oxidation stabilizer satisfies the above range, it is possible to effectively prevent a decrease in intrinsic viscosity that may occur in a subsequent process and to prevent a decrease in physical properties such as impact strength as well.

The branching agent may comprise at least one selected from the group consisting of trimellitic anhydride, trimethylol propane, and trimellitic acid.

In addition, the polyester resin may comprise the branching agent in an amount of 100 ppm to 2,500 ppm based on the total weight of the polyester resin. For example, the branching agent may be employed in an amount of 200 ppm to 2,500 ppm, 300 ppm to 2,300 ppm, 500 ppm to 2,300 ppm, 1,000 ppm to 2,500 ppm, 1,500 ppm to 2,300 ppm, or 1,800 ppm to 2,100 ppm, based on the total weight of the polyester resin. As the content of the branching agent satisfies the above range, the intrinsic viscosity may be more effectively controlled within a specific range, so that such physical properties as impact strength may be enhanced as well.

### Article

The article according to another embodiment of the present invention comprises the polyester resin.

The article may be obtained by molding a resin comprising the polyester resin using a molding method such as extrusion and injection. It may be a film (or sheet) or a component used in the fields of automobiles, electricity, and electronics.

In addition, the article may comprise labels or cap seals for various containers such as plastic, or packaging supplies.

### Process for preparing a polyester resin

The process for preparing a polyester resin according to another embodiment of the present invention comprises conducting alcoholysis and hydrolysis of waste polyester to prepare recycled terephthalic acid; mixing the recycled terephthalic acid with a glycol component to prepare a preliminary composition; subjecting the preliminary composition to an esterification reaction; and subjecting the esterification reaction product to a polycondensation reaction, wherein the recycled terephthalic acid has a Color b of less than 2.

### Step of conducting alcoholysis and hydrolysis of waste polyester to prepare recycled terephthalic acid

The process for preparing a polyester resin according to another embodiment of the present invention comprises conducting alcoholysis and hydrolysis of waste polyester to prepare recycled terephthalic acid.

Specifically, the step of preparing recycled terephthalic acid may comprise (1) subjecting waste polyester to alcoholysis with an alcohol having 4 or more carbon atoms to prepare a liquid composition comprising a compound represented by the following Formula 1; and (2) subjecting the liquid composition to hydrolysis.

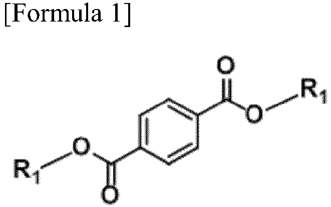

In Formula 1, R₁ is an alkyl group having 4 or more carbon atoms.

### Alcoholysis step (1)

The process for preparing recycled terephthalic acid according to another embodiment of the present invention comprises subjecting waste polyester to alcoholysis with an alcohol having 4 or more carbon atoms to prepare a liquid composition comprising a compound represented by Formula 1.

The waste polyester may be obtained by crushing or melting waste polyester products. For example, the waste polyester may be obtained by crushing polyester products that have been used up, recovered, and separated, or converting them into pellets (post-consumer recycled material; PCR), or polyester waste (post-industrial recycled material; PIR) such as defective products or scraps that may be formed during processes such as molding of polyester films, fibers, containers, or the like, but it is not limited thereto.

In addition, the number of carbon atoms of the alcohol may be 4 or more, 6 or more, 8 or more, 10 or more, or 12 or more, and may be 4 to 14, 4 to 13, 4 to 10, 4 to 8, 6 to 12, 8 to 14, or 8 to 13.

As the alcoholysis of waste polyester is carried out using an alcohol having the above number of carbon atoms, the alcoholysis can be carried out at a lower temperature and a lower pressure as compared with the conventional process using waste polyester that is carried out at a high temperature and a high pressure; and an intermediate, that is, an alcoholysis reaction product, can be produced in a liquid form. In addition, as the number of carbon atoms of the alcohol satisfies the above range, the alcoholysis reaction rate can be enhanced.

In addition, the alcohol may have a boiling point of 100°C to 290°C. For example, the boiling point of the alcohol may be 100°C to 280°C, 100°C to 260°C, 100°C to 230°C, 110°C to 190°C, or 180°C to 290°C. As the boiling point of the alcohol satisfies the above range, ethylene glycol formed as a by-product in the alcoholysis can be more easily removed or recovered in the subsequent process, thereby further enhancing processability. In particular, there has been a trend in recent years to use various monomer materials in the field in which polyester is used as a raw material; thus, it can be readily adopted for removing various dialcohol-type monomers used in waste polyester products such as waste plastic products.

The weight ratio of the waste polyester to the alcohol may be 1:1 to 10. For example, the weight ratio of the waste polyester to the alcohol may be 1:1 to 8, 1:1 to 6, 1:1 to 4, 1:1 to 3.5, 1:1.1 to 3.3, 1:2 to 4, or 1:2 to 3.5.

In addition, the alcoholysis reaction may be carried out at a temperature of 160°C to 280°C and a pressure of 1 bar to 40 bar for 0.5 hour to 24 hours. For example, the alcoholysis reaction may be carried out at a temperature of 165°C to 280°C, 165°C to 270°C, 180°C to 270°C, 190°C to 250°C, 200°C to 265°C, 220°C to 265°C, 240°C to 260°C, or 245°C to 260°C and a pressure of 1 bar to 38 bar, 1 bar to 33 bar, 1 bar to 28 bar, 1 bar to 24 bar, 2 bar to 40 bar, 3 bar to 35 bar, or 5 bar to 30 bar for 0.5 hour to 22 hours, 1 hour to 15 hours, 1.5 hours to 10 hours, 2 hours to 8 hours, or 2 hours to 6 hours.

In step (1), an alcoholysis catalyst may be added. Specifically, the alcoholysis reaction can be smoothly carried out as a non-catalytic reaction without using an alcoholysis catalyst, making it environmentally friendly. In particular, when the content of insoluble metals in waste polyester is high, a non-catalytic reaction may be advantageous for efficient treatment and removal of impurities. In addition, an alcoholysis catalyst may be added in step (1) from the viewpoint of energy to enhance processability by improving reactivity.

The alcoholysis catalyst may be a metal acetate salt, an alkali metal salt, or a hydroxy salt.

More specifically, the alcoholysis catalyst may comprise at least one cation selected from the group consisting of alkali metal ions such as Li⁺, Na⁺, K⁺, and Cs⁺, alkali earth metal ions such as Be²⁺, Mg²⁺, Ca²⁺, and Ba²⁺, ammonium ions such as NH⁴⁺ and NR⁴+ (where R is alkyl), and Zn²⁺; or at least one anion selected from the group consisting of OH⁻, OR⁻ (where R is alkyl), HCO₃⁻, CO₃²⁻, benzoate ion (C₇H₅O₂⁻), 4-alkoxycarbonylbenzoate ion, acetate ion, and terephthalate ion. R may be an alkyl group with 1 to 10 carbon atoms or an alkyl group with 1 to 5 carbon atoms.

For example, the alcoholysis catalyst may comprise at least one selected from the group consisting of Zn(OAC)₂, Co(OAc)₂, Mn(OAc)₂, Mg(OAc)₂, Ca(OAc)₂, Ba(OAc)₂, LiOAc, NaOAc, KOAc, Zn(OAC)₂·2H₂O, Co(OAc)₂·4H₂O, Pb(OAc)₂, Mn(OAc)₂·4H₂O, Mg(OAc)₂·4H₂O, Pd(OAc)₂, Ti(OBu)₄, Ti(OiPr)₄, GeO₂, Al(OiPr)₃, Na₂CO₃, K₂CO₃, dibutyltin(IV) oxide, stannous octoate, titanium phosphate, and terephthalic acid.

In addition, the amount of the alcoholysis catalyst added may be 10 ppm to 10,000 ppm based on the total weight of the waste polyester. For example, the amount of the alcoholysis catalyst added may be 10 ppm to 9,000 ppm, 15 ppm to 8,000 ppm, 20 ppm to 6,000 ppm, 50 ppm to 3,500 ppm, 100 ppm to 1,500 ppm, 150 ppm to 1,000 ppm, 180 ppm to 500 ppm, or 200 ppm to 450 ppm, based on the total weight of the waste polyester.

According to an embodiment of the present invention, a liquid composition comprising a compound represented by the following Formula 1 is prepared by the alcoholysis. Specifically, the composition is liquid at room temperature, and the liquid composition is a composition produced through an alcoholysis reaction.

The liquid composition may comprise a compound represented by the following Formula 1.

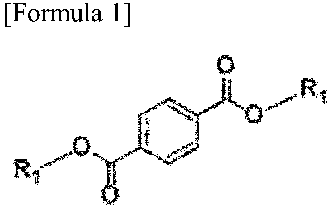

In Formula 1, R₁ is an alkyl group having 4 or more carbon atoms.

Specifically, R₁ may be butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, hexyl, 1-methylhexyl, 2-ethyl-1-hexyl, heptyl, n-heptyl, 1-methylheptyl, octyl, n-octyl, isooctyl, tert-octyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, decanyl, undecanyl, dodecanyl, tridecanyl, or tetradecanyl.

Specifically, the liquid composition may comprise unreacted alcohol and ethylene glycol (EG) as a by-product. More specifically, the liquid composition may comprise the compound represented by Formula 1, ethylene glycol (EG) formed as a by-product by the alcoholysis reaction, unreacted alcohol, and oligomers.

For example, the liquid composition may comprise a compound represented by the following Formula 2, and the oligomer may comprise a compound represented by following Formula 3.

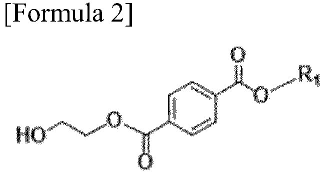

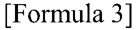

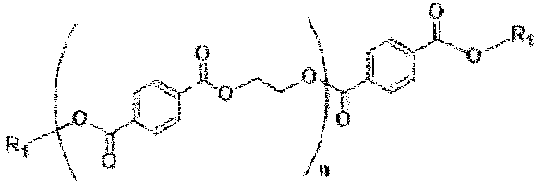

In Formulae 2 and 3, R₁ is an alkyl group having 4 or more carbon atoms, and n is an integer of 1 or more.

According to an embodiment of the present invention, the liquid composition may comprise unreacted alcohol and ethylene glycol as a by-product, and the content of the compound represented by Formula 1 in the liquid composition may be 70% by mole or more. For example, the content of the compound represented by Formula 1 in the liquid composition prepared by the alcoholysis may be 72% by mole or more, 75% by mole or more, 80% by mole or more, 85% by mole or more, 90% by mole or more, 91% by mole or more, 92% by mole or more, 92.5% by mole or more, 93% by mole or more, 95% by mole or more, 97% by mole or more, 99% by mole or more, or 99.5% by mole or more.

In addition, the content of oligomers in the liquid composition may be 15% by mole or less. For example, the content of oligomers in the liquid composition may be 11% by mole or less, 8.5% by mole or less, 7% by mole or less, 5% by mole or less, 3% by mole or less, 1% by mole or less, 0.8% by mole or less, 0.4% by mole or less, or 0.1% by mole or less.

According to another embodiment of the present invention, step (1) may comprise discharging the unreacted alcohol and ethylene glycol as a by-product.

Specifically, the alcohol is separated from the discharged mixture of alcohol and ethylene glycol, and the separated alcohol may be recycled as a raw material for the alcoholysis. For example, ethylene glycol as a by-product formed in the alcoholysis and unreacted alcohol (alcohol present in excess) are discharged in the form of a gaseous mixture in real time during the alcoholysis reaction and then subjected to fractional distillation or layer separation and/or to fractional distillation upon completion of the reaction to be separated into alcohol and ethylene glycol. The separated alcohol may be introduced into the alcoholysis in step (1) and reused. In such an event, the volume and feed rate of the alcohol introduced for reuse may be the same as the volume and discharge rate of the mixture of alcohol and ethylene glycol discharged.

In addition, step (1) may comprise recovering ethylene glycol as a by-product of the alcoholysis. Specifically, ethylene glycol as a by-product of the alcoholysis may be recovered by fractional distillation or layer separation of the mixture of alcohol and ethylene glycol discharged and may be recovered by fractional distillation or layer separation of the composition prepared in step (1).

For example, the unreacted alcohol and ethylene glycol as a by-product are discharged as a gaseous mixture in real time during the alcoholysis reaction, which is condensed using an external cooling apparatus to recover ethylene glycol.

According to an embodiment of the present invention, not only can unreacted alcohol and ethylene glycol be separated through a simple process such as fractional distillation or layer separation, but the separated unreacted alcohol can also be recycled as a raw material for the alcoholysis, and the recovered ethylene glycol can also be used in other processes. Thus, processability and process cost reduction are excellent.

The recovery rate of ethylene glycol may be 65% or more. For example, the recovery rate of ethylene glycol may be 70% or more, 76% or more, 85% or more, 90% or more, 93% or more, 95% or more, 97% or more, 98% or more, 99% or more, or 99.5% or more.

### Purification step

The process for preparing recycled terephthalic acid according to another embodiment of the present invention may further comprise purifying the liquid composition before step (2) to be described below.

Specifically, the purification step may comprise adding at least one adsorbent selected from the group consisting of activated carbon, silica gel, alumina, zeolite, and activated clay, or adsorption through bed adsorption. More specifically, the adsorbent may be activated carbon or a mixture of activated carbon and silica gel. For example, the adsorbent may be a mixture of activated carbon and silica gel at a weight ratio of 1:0.5 to 1.5 or 1:0.8 to 1.2, but it is not limited thereto.

The content of the adsorbent added may be 0.1% by weight to 20% by weight based on the total weight of the liquid composition. For example, the content of the adsorbent added may be 0.1% by weight to 18% by weight, 0.1% by weight to 15% by weight, 0.1% by weight to 10% by weight, 0.1% by weight to 5% by weight, or 0.1% by weight to 2% by weight, based on the total weight of the liquid composition.

As the step of purifying the liquid composition using the adsorbent, specifically using the adsorbent that satisfies the specific feed amount within the above numerical range, is further carried out, the purity and yield can be further enhanced. Specifically, as the step of purifying the liquid composition, which is the alcoholysis reaction composition, is carried out, it is possible to more effectively remove insoluble impurities such as metals that may be contained in the alcoholysis reaction composition, or additives such as colorants and pigments that may be contained in waste polyester. Thus, the purity and yield of the terephthalic acid finally prepared can be further enhanced.

In addition, the process for preparing recycled terephthalic acid according to another embodiment of the present invention may further comprise a concentration step after the purification step.

The concentration may be carried out at a temperature of 50°C to 120°C for 0.5 hour to 6 hours. For example, the concentration may be carried out at a temperature of 55°C to 115°C, 60°C to 110°C, 65°C to 105°C, or 75°C to 100°C for 1 hour to 5 hours, 1.5 hours to 4 hours, or 2 hours to 4 hours by stirring the purified alcoholysis reaction composition.

In addition, the purified composition may have a pigment residual rate (%) of 15% or less according to the following Equation A. [Equation A] $Pigment residual rate of recycled terephthalic acid \left(%\right) = \frac{B 1}{B 2} \times 100$

In Equation A, A1 is the area of an absorbance curve obtained in 400 nm to 800 nm using a UV-vis spectrophotometer for the purified alcoholysis reaction composition as diluted to a concentration of 5% in dimethyl sulfoxide (DMSO), dimethyl formamide (DMF), or methylpyrrolidone (NMP), and A2 is the area of an absorbance curve obtained in the same manner as above for the alcoholysis reaction composition that has not been purified.

For example, the pigment residual rate (%) of the purified alcoholysis reaction composition according to Equation A may be 13% or less, 11% or less, 10% or less, 8% or less, 6% or less, 5.5% or less, 5% or less, 4.3% or less, or 4% or less.

As a specific example, when dimethyl sulfoxide (DMSO) is used as a solvent for diluting the alcoholysis reaction composition, the pigment residual rate according to the above Equation A may be 11% or less, 10% or less, or 8.5% or less. When dimethylformamide (DMF) is used, the pigment residual according to the above Equation A may be 10.5% or less, 9% or less, or 8.3% or less. When methylpyrrolidone (NMP) is used, the pigment residual according to the above Equation A may be 10% or less, 8.5% or less, or 8% or less.

### Hydrolysis reaction step (2)

The process for preparing recycled terephthalic acid according to an embodiment of the present invention comprises subjecting the liquid composition to hydrolysis. Specifically, in step (2), the alcoholysis reaction composition (liquid composition comprising the compound represented by the above Formula 1) prepared in step (1), or the purified alcoholysis reaction composition, may be subjected to hydrolysis to prepare recycled terephthalic acid.

The hydrolysis may be carried out by adding water to the composition. For example, the hydrolysis may be carried out by adding water to the alcoholysis reaction composition, or the purified alcoholysis reaction composition, at a temperature of 180°C to 280°C, 185°C to 280°C, 200°C to 275°C, 220°C to 270°C, or 240°C to 265°C, for 0.5 hour to 24 hours, 1 hour to 20 hours, 2.5 hours to 12 hours, or 3 hours to 8 hours.

The conventional method in which a metal catalyst such as iron, cobalt, manganese, or nickel is added to waste polyester, which is then directly hydrolyzed with water, is environmentally friendly. However, processability is low in that very high-temperature conditions of 300°C or higher are required, and the reaction apparatus must also have high-pressure resistance. In contrast, the process for preparing recycled terephthalic acid according to an embodiment of the present invention has excellent processability since the process conditions are ameliorated as compared with the prior art.

In addition, the weight ratio of the composition to water may be 1:1 to 500. For example, the weight ratio of the alcoholysis reaction composition (purified, or purified and concentrated, alcoholysis reaction composition) used in the hydrolysis to water may be 1:1 to 450, 1:1 to 400, 1:1 to 250, 1:1 to 100, 1:1 to 50, 1:1.2 to 20, or 1:1.5 to 10.

In addition, in step (2), a hydrolysis catalyst may be added. Specifically, a hydrolysis catalyst may be added to the mixture of the alcoholysis reaction composition and water to carry out hydrolysis.

The hydrolysis reaction can be smoothly carried out as a non-catalytic reaction without using a hydrolysis catalyst, making it environmentally friendly. In particular, when the content of insoluble metals in waste polyester is high, a non-catalytic reaction may be advantageous for efficient treatment and removal of impurities. In addition, a hydrolysis catalyst may be added in step (2) from the viewpoint of energy to enhance processability by improving reactivity.

The hydrolysis catalyst may be a metal acetate salt, an alkali metal salt, or a hydroxy salt.

More specifically, the hydrolysis catalyst may comprise at least one cation selected from the group consisting of alkali metal ions such as Li⁺, Na⁺, K⁺, and Cs⁺, alkali earth metal ions such as Be²⁺, Mg²⁺, Ca²⁺, and Ba²⁺, ammonium ions such as NH⁴⁺ and NR⁴⁺ (where R is alkyl), and Zn²⁺; or at least one anion selected from the group consisting of OH⁻, OR⁻ (where R is alkyl), HCO₃⁻, CO₃²⁻, benzoate ion (C₇H₅O₂⁻), 4-alkoxycarbonylbenzoate ion, acetate ion, and terephthalate ion. R may be an alkyl group with 1 to 10 carbon atoms or an alkyl group with 1 to 5 carbon atoms.

For example, the hydrolysis catalyst may comprise at least one selected from the group consisting of Zn(OAC)₂, Co(OAc)₂, Mn(OAc)₂, Mg(OAc)₂, Ca(OAc)₂, Ba(OAc)₂, LiOAc, NaOAc, KOAc, Zn(OAC)₂·2H₂O, Co(OAc)₂·4H₂O, Pb(OAc)₂, Mn(OAc)₂·4H₂O, Mg(OAc)₂·4H₂O, Pd(OAc)₂, Ti(OBu)₄, Ti(OiPr)₄, GeO₂, Al(OiPr)₃, Na₂CO₃, K₂CO₃, dibutyltin(IV) oxide, stannous octoate, titanium phosphate, and terephthalic acid.

In addition, the amount of the hydrolysis catalyst added may be 10 ppm to 10,000 ppm based on the total weight of the composition. For example, the amount of the hydrolysis catalyst added may be 15 ppm to 8,000 ppm, 20 ppm to 5,500 ppm, 30 ppm to 3,000 ppm, 50 ppm to 1,600 ppm, 100 ppm to 1,200 ppm, 150 ppm to 1,100 ppm, 300 ppm to 1,000 ppm, 350 ppm to 950 ppm, 400 ppm to 850 ppm, 420 ppm to 700 ppm, or 450 ppm to 650 ppm, based on the total weight of the alcoholysis reaction composition (purified, or purified and concentrated, alcoholysis reaction composition).

According to an embodiment of the present invention, solid recycled terephthalic acid may be prepared through the hydrolysis reaction. Specifically, the process may further comprise, after the hydrolysis step, filtering, washing, and drying the hydrolysis reaction product prepared by the hydrolysis reaction. That is, the hydrolysis reaction product prepared through the hydrolysis reaction may be filtered, washed, and dried to produce solid recycled terephthalic acid.

For example, the hydrolysis reaction product may be cooled to an appropriate temperature, for example, from room temperature to lower than 100°C, at which water does not vaporize, to obtain a solution in the form of a slurry, which is filtered to obtain a solid, which is washed and dried under vacuum to obtain solid recycled terephthalic acid.

The washing may be carried out using a mixture of an alcohol having 4 or more carbon atoms and/or water, a protic solvent such as isopropanol and acetic acid, or an aprotic solvent such as acetone, dichloromethane, chloroform, tetrahydrofuran (THF), and toluene.

Here, the description on the alcohol having 4 or more carbon atoms is as described above. The alcohol with 4 or more carbon atoms used in the washing may be the same as, or different from, the alcohol with 4 or more carbon atoms used in step (1). For example, when 1-butanol is used as alcohol in step (1) for producing terephthalic acid, the solid terephthalic acid prepared may be washed with a mixture of 1-butanol and water. In addition, the number of carbon atoms in the alcohol used in the washing may be the same as the number of carbon atoms in the alcohol used in step (1).

Residual pigments or yellow impurities generated by the decomposition of pigments during hydrolysis can be effectively removed through the washing, thereby enhancing the yellow index or color characteristics. In addition, as water is used for washing, mineral salts can be removed, thereby enhancing quality.

In addition, the yield of recycled terephthalic acid may be 65% or more. For example, the yield of the recycled terephthalic acid finally produced may be 68% or more, 70% or more, 75% or more, 80% or more, 85% or more, 90% or more, or 95% or more.

### Step of mixing the recycled terephthalic acid with a glycol component to prepare a preliminary composition

The process for preparing a polyester resin according to another embodiment of the present invention comprises mixing the recycled terephthalic acid with a glycol component to prepare a preliminary composition.

In addition, a diacid component may be further mixed in the step of preparing the preliminary composition. Details on the diacid component and the glycol component are as described above.

In addition, at least one additive selected from the group consisting of a colorant, a crystallizing agent, an oxidation stabilizer, and a branching agent may be further added in the step of preparing the preliminary composition. Details on the additive are as described above.

### Step of subjecting the preliminary composition to an esterification reaction

The process for preparing a polyester resin according to another embodiment of the present invention may further comprise subjecting the preliminary composition to an esterification reaction.

The esterification reaction may be carried out at a temperature of 200°C to 350°C, 220°C to 320°C, or 250°C to 290°C. In addition, the esterification reaction may be carried out in a pressurized state higher than normal pressure by 0 kg/cm² to 10 kg/cm² (0 mmHg to 7,355.6 mmHg), 0 kg/cm² to 5 kg/cm² (0 to 3,677.8 mmHg) or 0 kg/cm² to 2.0 kg/cm² (0 to 1,471.1 mmHg). In addition, the esterification reaction may be carried out for 1 hour to 24 hours, 1 hour to 10 hours, or 1 hour to 6 hours.

### Step of subjecting the esterification reaction product to a polycondensation reaction

The process for preparing a polyester resin according to another embodiment of the present invention comprises subjecting the esterification reaction product to a polycondensation reaction.

In addition, the polycondensation reaction may be carried out at a temperature of 150°C to 400°C, 200°C to 370°C, 250°C to 350°C, or 270°C to 300°C. In addition, the polycondensation reaction may be carried out at a reduced pressure of 0.01 mmHg to 400 mmHg, 0.05 mmHg to 100 mmHg, or 0.1 mmHg to 100 mmHg. In addition, the polycondensation reaction may be carried out for the required time until the desired intrinsic viscosity is reached. For example, it may be carried out for 1 hour to 24 hours, 1 hour to 10 hours, or 1 hour to 4 hours.

At the beginning of the polycondensation reaction, the stirring speed may be set high. As the polycondensation reaction proceeds, when the stirring power is weakened due to the increase in the viscosity of the reactant or the temperature of the reactant rises above the set temperature, the stirring speed may be appropriately adjusted accordingly.

In addition, a catalyst and/or a stabilizer may be further added in the esterification reaction and the polycondensation reaction.

For example, the catalyst for the esterification reaction may be methylates of sodium and magnesium; acetates, borates, fatty acid salts, and carbonates of Zn, Cd, Mn, Co, Ca, and Ba; metallic Mg; and oxides of Pb, Zn, Sb, Ge, and Ti.

In addition, the catalyst for a polycondensation reaction may be, for example, titanium-based catalysts such as tetraethyl titanate, acetyl tripropyl titanate, tetrapropyl titanate, tetrabutyl titanate, polybutyl titanate, 2-ethylhexyl titanate, octylene glycol titanate, lactate titanate, triethanolamine titanate, acetyl acetonate titanate, ethyl acetoacetic ester titanate, isostearyl titanate, titanium dioxide, titanium dioxide/silicon dioxide copolymers, titanium dioxide/zirconium dioxide copolymers; germanium-based catalysts such as germanium dioxide and copolymers using the same; or tin-based catalysts such as monobutyltin oxide, dibutyltinoxide, and monobutylhydroxytinoxide.

In addition, the stabilizer may be a phosphorus-based compound such as phosphoric acid, trimethyl phosphate, and triethyl phosphate may be used, but it is not limited thereto.

The process for preparing a polyester resin according to another embodiment of the present invention may further comprises carrying out a solid-state polymerization reaction. For example, the solid-state polymerization may be carried out after the polycondensation reaction at a temperature of 190°C to 230°C under vacuum conditions of 0.2 Torr to 2.0 Torr or a nitrogen atmosphere.

Hereinafter, the present invention will be described in more detail with reference to the following examples. However, these examples are set forth to illustrate the present invention, and the scope of the present invention is not limited thereto.

### Embodiments for Carrying Out the Invention

### [Example]

### Preparation of a liquid composition

### Example 1-1

A first high-pressure reactor with a capacity of 7 liters was charged with 1 kg of waste polyethylene terephthalate (waste PET) and 3.3 kg of 1-butanol as an alcohol, followed by an addition thereto of 200 mg of Zn(OAC)₂·2H₂O as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 24 bar were maintained.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by the following Formula 1 (R₁: (CH₂)₃CH₃), residual ethylene glycol (EG), an alcohol (1-butanol), and oligomers.

Thereafter, the alcoholysis reaction composition was charged to a separate flask, and excess unreacted 1-butanol and produced ethylene glycol (EG) were recovered using a fractional distillation apparatus, respectively.

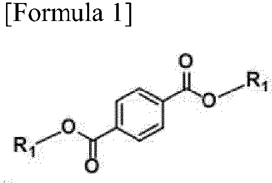

### Example 1-2

A first high-pressure reactor with a capacity of 7 liters was charged with 1 kg of waste polyethylene terephthalate (waste PET) and 3.3 kg of 1-butanol as an alcohol, followed by an addition thereto of 200 mg of Zn(OAC)₂·2H₂O as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 24 bar were maintained.

Specifically, one hour after the alcoholysis reaction began, the valve of the back pressure regulator installed in advance was adjusted to discharge a gaseous mixture of ethylene glycol (EG), a by-product produced by the alcoholysis reaction, and 1-butanol present in excess. In such an event, the internal temperature of the first high-pressure reactor was maintained at 250°C, and the gaseous mixture of ethylene glycol (EG) and 1-butanol discharged through the back pressure regulator was condensed using an external cooling device. In addition, the discharge rate of the gaseous mixture of ethylene glycol (EG) and 1-butanol was adjusted to 3 kg/h, and, at the same time, 1-butanol was continuously supplied to the first high-pressure reactor. In such an event, the volume and feed rate of 1-butanol newly supplied to the first high-pressure reactor were adjusted to be the same as the volume and discharge rate of the gaseous mixture of ethylene glycol (EG) and 1-butanol discharged. An alcoholysis reaction was carried out while the discharge and feed processes were maintained for 3 hours.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by the following Formula 1 (R₁: (CH₂)₃CH₃), residual ethylene glycol (EG), an alcohol (1-butanol), and oligomers.

Thereafter, the alcoholysis reaction composition was charged to a separate flask, and excess unreacted 1-butanol and produced ethylene glycol were recovered using a fractional distillation apparatus, respectively.

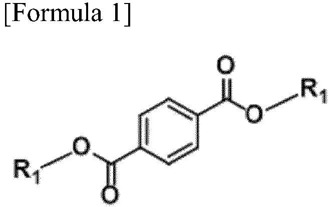

### Example 1-3

A first high-pressure reactor with a capacity of 7 liters was charged with 1 kg of waste polyethylene terephthalate (waste PET) and 3.3 kg of 1-butanol as an alcohol, followed by an addition thereto of 200 mg of Zn(OAC)₂·2H₂O as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 24 bar were maintained.

Specifically, one hour after the alcoholysis reaction began, the valve of the back pressure regulator installed in advance was adjusted to discharge a gaseous mixture of ethylene glycol (EG), a by-product produced by the alcoholysis reaction, and 1-butanol present in excess. In such an event, the internal temperature of the first high-pressure reactor was maintained at 250°C, and the gaseous mixture of ethylene glycol (EG) and 1-butanol discharged through the back pressure regulator was condensed using an external cooling device. In addition, the discharge rate of the gaseous mixture of ethylene glycol (EG) and 1-butanol was adjusted to 3 kg/h, and, at the same time, 1-butanol was continuously supplied to the first high-pressure reactor. In such an event, the volume and feed rate of 1-butanol newly supplied to the first high-pressure reactor were adjusted to be the same as the volume and discharge rate of the gaseous mixture of ethylene glycol (EG) and 1-butanol discharged.

The condensed gaseous mixture of ethylene glycol (EG) and 1-butanol was transferred to a layer separation apparatus with a capacity of 5 liters filled with 3 kg of water, and a procedure of stirring for 10 minutes and layer separation for 2 minutes was repeated to separate a 1-butanol layer and a water and ethylene glycol (EG) layer. The separated 1-butanol was fed to the first high-pressure reactor again in real time using a high-pressure pump. An alcoholysis reaction was carried out while the procedure of stirring, layer separation, and reintroduction was maintained for 3 hours.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by the following Formula 1 (R₁: (CH₂)₃CH₃), residual ethylene glycol (EG), an alcohol (1-butanol), and oligomers.

Thereafter, the alcoholysis reaction composition was charged to a separate flask, and excess unreacted 1-butanol and produced ethylene glycol were recovered using a fractional distillation apparatus, respectively.

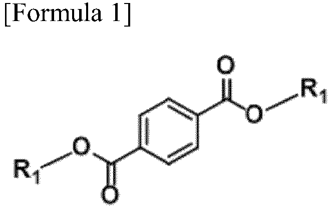

### Example 1-4

A liquid alcoholysis reaction composition was prepared in the same manner as in Example 1-1, except that 3.3 kg of 1-pentanol was used as an alcohol, and the pressure was maintained at 13 bar.

### Example 1-5

A liquid alcoholysis reaction composition was prepared in the same manner as in Example 1-2, except that 3.3 kg of 1-pentanol was used as an alcohol, and the pressure was maintained at 13 bar.

### Example 1-6

A liquid alcoholysis reaction composition was prepared in the same manner as in Example 1-3, except that 3.3 kg of 1-pentanol was used as an alcohol, and the pressure was maintained at 13 bar.

### Example 1-7

A liquid alcoholysis reaction composition was prepared in the same manner as in Example 1-1, except that 3.3 kg of 1-octanol was used as an alcohol, and the pressure was maintained at 3.4 bar.

### Example 1-8

A liquid alcoholysis reaction composition was prepared in the same manner as in Example 1-2, except that 3.3 kg of 1-octanol was used as an alcohol, and the pressure was maintained at 3.4 bar.

### Example 1-9

A liquid alcoholysis reaction composition was prepared in the same manner as in Example 1-3, except that 3.3 kg of 1-octanol was used as an alcohol, and the pressure was maintained at 3.4 bar.

### Example 1-10

A liquid alcoholysis reaction composition was prepared in the same manner as in Example 1-1, except that 3.3 kg of 2-ethyl-1-hexanol was used as an alcohol, and the pressure was maintained at 4.1 bar.

### Example 1-11

A liquid alcoholysis reaction composition was prepared in the same manner as in Example 1-2, except that 3.3 kg of 2-ethyl-1-hexanol was used as an alcohol, and the pressure was maintained at 4.1 bar.

### Example 1-12

A liquid alcoholysis reaction composition was prepared in the same manner as in Example 1-3, except that 3.3 kg of 2-ethyl-1-hexanol was used as an alcohol, and the pressure was maintained at 4.1 bar.

### Example 1-13

A liquid alcoholysis reaction composition was prepared in the same manner as in Example 1-1, except that 3.3 kg of 1-decanol was used as an alcohol, and the pressure was maintained at 1.6 bar.

### Example 1-14

A liquid alcoholysis reaction composition was prepared in the same manner as in Example 1-2, except that 3.3 kg of 1-decanol was used as an alcohol, and the pressure was maintained at 1.6 bar.

### Example 1-15

A liquid alcoholysis reaction composition was prepared in the same manner as in Example 1-3, except that 3.3 kg of 1-decanol was used as an alcohol, and the pressure was maintained at 1.6 bar.

### Example 1-16

A liquid alcoholysis reaction composition was prepared in the same manner as in Example 1-1, except that 3.3 kg of 1-dodecanol was used as an alcohol, and the pressure was maintained at 1.0 bar.

### Example 1-17

A first high-pressure reactor with a capacity of 7 liters was charged with 1 kg of waste polyethylene terephthalate (waste PET) and 3.3 kg of 1-dodecanol as an alcohol, followed by an addition thereto of 200 mg of Zn(OAC)₂·2H₂O as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 1.0 bar were maintained.

Specifically, one hour after the alcoholysis reaction began, the valve of the back pressure regulator installed in advance was adjusted to discharge the vapor of ethylene glycol (EG), a by-product produced by the alcoholysis reaction. In such an event, the internal temperature of the first high-pressure reactor was maintained at 250°C, and the vapor of ethylene glycol (EG) was condensed using an external cooling device.

In addition, 1-dodecanol was continuously supplied to the first high-pressure reactor in an amount equal to the amount of ethylene glycol (EG) discharged as vapor and condensed. In such an event, the volume and feed rate of 1-dodecanol newly supplied to the first high-pressure reactor were adjusted to be the same as the volume and discharge rate of the vapor of ethylene glycol (EG) discharged. An alcoholysis reaction was carried out while the discharge and feed processes were maintained for 3 hours.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by the following Formula 1 (R₁: (CH₂)₁₁CH₃), residual ethylene glycol (EG), an alcohol (1-dodecanol), and oligomers.

Thereafter, the alcoholysis reaction composition was charged to a separate flask, and excess unreacted 1-dodecanol and produced ethylene glycol (EG) were recovered using a fractional distillation apparatus, respectively.

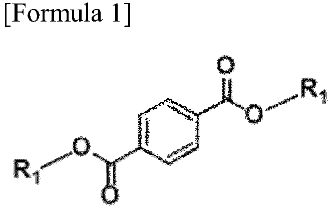

### Example 1-18

A liquid alcoholysis reaction composition was prepared in the same manner as in Example 1-1, except that 3.3 kg of 1-tetradecanol was used as an alcohol, and the pressure was maintained at 1.0 bar.

### Example 1-19

A liquid alcoholysis reaction composition was prepared in the same manner as in Example 1-17, except that 3.3 kg of 1-tetradecanol was used as an alcohol.

### Comparative Example 1-1

A liquid alcoholysis reaction composition was prepared in the same manner as in Example 1-1, except that 3.3 kg of methanol was used as an alcohol, and the pressure was maintained at 83 bar.

### Comparative Example 1-2

A liquid alcoholysis reaction composition was prepared in the same manner as in Example 1-2, except that 3.3 kg of methanol was used as an alcohol, and the pressure was maintained at 83 bar.

### Comparative Example 1-3

A liquid alcoholysis reaction composition was prepared in the same manner as in Example 1-1, except that 3.3 kg of ethanol was used as an alcohol, and the pressure was maintained at 64 bar.

### Comparative Example 1-4

A liquid alcoholysis reaction composition was prepared in the same manner as in Example 1-2, except that 3.3 kg of ethanol was used as an alcohol, and the pressure was maintained at 64 bar.

**[Table 1]**

| | | Liquid composition | | | | Recovery rate of EG (%) | Process pressure (bar) |
|---|---|---|---|---|---|---|---|
| | | Compound of Formula 1 | | EG and alcohol (% by mole) | Oligomer (% by mole) | | |
| | | R₁ | Content (% by mole) | | | | |
| Ex. 1-1 | 1-butanol | -(CH₂)₃CH₃ | 91.3 | 8.1 | 0.6 | 94.0 | 24 |
| Ex. 1-2 | | | 93.2 | 6.2 | 0.6 | 95.1 | 24 |
| Ex. 1-3 | | | 92.7 | 6.6 | 0.7 | 96.2 | 24 |
| Ex. 1-4 | 1-pentanol | -(CH₂)₄CH₃ | 92.1 | 7.4 | 0.5 | 97.5 | 13 |
| Ex. 1-5 | | | 97.3 | 2.4 | 0.3 | 93.3 | 13 |
| Ex. 1-6 | | | 97.8 | 2.0 | 0.2 | 98.1 | 13 |
| Ex. 1-7 | 1-octanol | -(CH₂)₇CH₃ | 93.3 | 6.1 | 0.6 | 92.1 | 3.4 |
| Ex. 1-8 | | | 99.5 | 0.4 | <0.1 | 99.6 | 3.4 |
| Ex. 1-9 | | | 99.6 | 0.3 | <0.1 | 99.7 | 3.4 |
| Ex. 1-10 | 2-ethyl-1-hax anol | CH₂CH(CH₂)₃CH CH₂CH₃ | 93.6 | 5.0 | 1.4 | 92.3 | 4.1 |
| Ex. 1-11 | | | 99.7 | 0.2 | <0.1 | 99.8 | 4.1 |
| Ex. 1-12 | | | 99.7 | 0.2 | <0.1 | 99.6 | 4.1 |
| Ex. 1-13 | 1-decanol | -(CH₂)₉CH₃ | 91.9 | 6.5 | 1.6 | 91.8 | 1.6 |
| Ex. 1-14 | | | 99.5 | 0.4 | <0.1 | 99.6 | 1.6 |
| Ex. 1-15 | | | 99.6 | 0.3 | <0.1 | 99.5 | 1.6 |
| Ex. 1-16 | 1-dodecanol | -(CH₂)₁₁CH₃ | 91.0 | 8.3 | 0.7 | 92.2 | 1.0 |
| Ex. 1-17 | | | 99.6 | 0.3 | <0.1 | 99.7 | 1.0 |
| Ex. 1-18 | 1-tetradecanol | -(CH₂)₁₃CH₃ | 90.0 | 8.1 | 1.9 | 93.3 | 1.0 |
| Ex. 1-19 | | | 99.6 | 0.3 | <0.1 | 99.6 | 1.0 |
| C. Ex. 1-1 | methanol | -CH₃ | 82.2 | 13.1 | 2.7 | 85.5 | 83 |
| C. Ex. 1-2 | methanol | -CH₃ | 85.1 | 12.3 | 2.6 | 84.2 | 83 |
| C. Ex. 1-3 | ethanol | -CH₂CH₃ | 77.5 | 13.0 | 9.5 | 80.0 | 64 |
| C. Ex. 1-4 | ethanol | -CH₂CH₃ | 80.2 | 15.5 | 4.3 | 78.3 | 64 |

As can be seen from Table 1 above, as the liquid alcoholysis reaction compositions of Examples 1-1 to 1-19 were prepared using an alcohol having 4 or more carbon atoms, they could be prepared at a very low process pressure as compared with Comparative Examples 1-1 to 1-4. The content, that is, the yield and purity, of the compound represented by Formula 1 was high, and the recovery rate of ethylene glycol was also excellent.

### Purification and concentration of the liquid composition

### Example 2-1

0.1 g of activated carbon as an adsorbent was added to 100 g of the liquid alcoholysis reaction composition before the excess unreacted alcohol and produced ethylene glycol were recovered using a fractional distillation apparatus in Example 1-1, followed by purification thereof, which was then stirred at 100°C for 3 hours and then filtered to concentrate the alcoholysis reaction composition.

### Examples 2-2 to 2-23 and Comparative Examples 2-1 and 2-2

The liquid alcoholysis reaction compositions were each purified and concentrated in the same manner as in Example 2-1, except that the liquid composition and process conditions were changed as shown in Table 2 below. Here, in Comparative Examples 2-1 and 2-2, since it was precipitated in an undissolved state, it was not purified.

### Examples 2-24 to 2-26

0.1 g of activated carbon as an adsorbent was added to 100 g of the liquid alcoholysis reaction compositions after the excess unreacted alcohol and produced ethylene glycol were recovered using a fractional distillation device in each of Examples 1-1, 1-4, and 1-10, followed by purification thereof, which was stirred at 100°C for 3 hours and then filtered to concentrate the alcoholysis reaction composition.

### Comparative Examples 2-3 to 2-21

An adsorbent was not added to the liquid alcoholysis reaction composition before the excess unreacted alcohol and produced ethylene glycol were recovered using a fractional distillation device in each of Examples 1-1 to 1-19, which was stirred at 100°C for 3 hours and then filtered to concentrate it.

### Test Example 1-1: Pigment residual rate

The compositions of Examples 2-1 to 2-26 and Comparative Examples 2-1 to 2-21 were each measured for pigment residual rate (%).

Specifically, the composition of Example 2-1 was diluted to a concentration of 5% in dimethyl sulfoxide (DMSO), dimethyl formamide (DMF), and methylpyrrolidone (NMP), respectively, and the area (A1) of an absorbance curve was obtained in 400 nm to 800 nm using a UV-vis spectrophotometer. In addition, for the composition of Comparative Example 2-3 (the composition of Example 1-1 was used in the same manner as the composition of Example 2-1 above whereas the composition was concentrated without purification by adding an adsorbent), the area (A2) of an absorbance curve was obtained in 400 nm to 800 nm in the same manner as above. The pigment residual rate (%) was calculated according to the following Equation A using the areas of the measured absorbance curve. [Equation A] $Pigment residual rate of recycled terephthalic acid \left(%\right) = \frac{B 1}{B 2} \times 100$

The compositions of Examples 2-2 to 2-26 and Comparative Examples 2-1 to 2-21 were each measured for pigment residual rate (%) in the same manner as above.

### Test Example 1-2: Yellow index

The compositions of Examples 2-1 to 2-26 and Comparative Examples 2-1 to 2-21 were each measured for yellow index (Y.I.).

Specifically, the compositions of Examples 2-1 to 2-26 and Comparative Examples 2-1 to 2-21 were each diluted to a concentration of 5% in a solvent, and the yellow index was measured using ColorFlex EZ (manufacturer: HunterLab) equipment. In such an event, the solvent used was dimethyl sulfoxide (DMSO), dimethyl formamide (DMF), or methyl pyrrolidone (NMP).

### Test Example 1-3: Content of metals

The content (ppm) of metals present in each of the compositions of Examples 2-1 to 2-26 and Comparative Examples 2-1 to 2-21 was measured using inductively coupled plasma atomic emission spectroscopy (ICP-AES). Here, N.D. indicates that the content was too low, less than 1 ppm, to be measured as a specific value.

| [Table 2] | | | | | | |
|---|---|---|---|---|---|---|
| | Comp. | Process conditions | | Pigment residual rate (%) DMSO/DMF/NMP | Y.I. DMSO/DMF/ NMP | Metal content (ppm) (Sb/Ti/Zn) |
| Ex. 2-1 | Ex. 1-1 | Activated carbon 0.1g | 100°C/3h | 5.4/6.1/5.7 | 1.6/1.4/1.6 | 18/5/4 |
| Ex. 2-2 | Ex. 1-2 | Activated carbon 0.1g | 100°C/3h | 6.8/6.5/6.0 | 1.4/1.5/1.3 | 15/6/3 |
| Ex. 2-3 | Ex. 1-3 | Activated carbon 0.1g | 100°C/3h | 7.6/7.5/6.9 | 1.6/1.7/1.3 | 12/2/2 |
| Ex. 2-4 | Ex. 1-4 | Activated carbon 0.1g | 100°C/3h | 8.1/8.3/7.8 | 1.8/1.5/1.6 | 15/4/3 |
| Ex. 2-5 | Ex. 1-5 | Activated carbon 0.1g | 100°C/3h | 7.8/8.0/7.9 | 1.7/1.7/1.3 | 18/2/2 |
| Ex. 2-6 | Ex. 1-6 | Activated carbon 0.1g | 100°C/3h | 6.2/6.0/6.5 | 1.5/1.2/1.0 | 16/4/2 |
| Ex. 2-7 | Ex. 1-7 | Activated carbon 0.1g | 100°C/3h | 5.3/5.3/5.8 | 1.5/1.2/1.4 | 18/4/3 |
| Ex. 2-8 | Ex. 1-8 | Activated carbon 0.1g | 100°C/3h | 6.5/5.5/6.1 | 1.1/1.3/1.6 | 19/2/3 |
| Ex. 2-9 | Ex. 1-9 | Activated carbon 0.1g | 100°C/3h | 6.7/5.8/6.0 | 1.3/1.3/1.5 | 21/2/3 |
| Ex. 2-10 | Ex. 1-10 | Activated carbon 0.1g | 100°C/3h | 7.5/6.1/6.8 | 1.4/1.3/1.6 | 20/3/3 |
| Ex. 2-11 | Ex. 1-11 | Activated carbon 0.1g | 100°C/3h | 6.0/5.3/6.1 | 1.3/1.4/1.3 | 17/2/3 |
| Ex. 2-12 | Ex. 1-11 | Activated carbon 0.05g | 100°C/3h | 7.9/7.1/7.8 | 1.8/1.8/1.6 | 21/2/3 |
| Ex. 2-13 | Ex. 1-11 | Activated carbon 0.5g | 100°C/3h | 5.114.8/4.0 | 0.9/1.0/0.8 | 15/2/2 |
| Ex. 2-14 | Ex. 1-11 | Activated carbon 0.1g /Silica gel 0.1g | 100°C/3h | 4.8/4.3/5.1 | 1.4/1.2/1.5 | N.D./ N.D./ N.D. |
| Ex. 2-15 | Ex. 1-11 | Activated carbon 0.05g /Silica gel 0.05g | 100°C/3h | 7.0/7.3/6.9 | 1.6/1.3/1.2 | N.D./ N.D./ N.D. |
| Ex. 2-16 | Ex. 1-12 | Activated carbon 0.1g | 100°C/3h | 5.6/5.3/5.8 | 1.1/1.1/1.3 | 19/4/2 |
| Ex. 2-17 | Ex. 1-13 | Activated carbon 0.1g | 100°C/3h | 6.1/5.9/5.5 | 1.2/1.0/1.4 | 17/3/2 |
| Ex. 2-18 | Ex. 1-14 | Activated carbon 0.1g | 100°C/3h | 8.1/7.9/7.5 | 0.9/1.1/1.4 | 15/4/3 |
| Ex. 2-19 | Ex. 1-15 | Activated carbon 0.1g | 100°C/3h | 7.7/7.2/6.7 | 1.0/1.0/1.3 | 19/3/2 |
| Ex. 2-20 | Ex. 1-16 | Activated carbon 0.1g | 100°C/3h | 7.4/6.9/6.1 | 1.0/1.3/1.6 | 13/3/3 |
| Ex. 2-21 | Ex. 1-17 | Activated carbon 0.1g | 100°C/3h | 6.6/6.0/6.2 | 1.2/1.5/1.7 | 16/3/2 |
| Ex. 2-22 | Ex. 1-18 | Activated carbon 0.1g | 100°C/3h | 7.7/7.2/6.7 | 1.7/1.0/1.2 | 15/2/4 |
| Ex. 2-23 | Ex. 1-19 | Activated carbon 0.1g | 100°C/3h | 7.7/7.2/6.7 | 1.1/1.1/1.3 | 11/3/2 |
| Ex. 2-24 | Ex. 1-1 | Activated carbon 0.1g | 100°C/3h | 5.1/5.0/4.9 | 1.5/1.5/1.6 | 15/4/4 |
| Ex. 2-25 | Ex. 1-4 | Activated carbon 0.1g | 100°C/3h | 8.4/8.2/7.8 | 1.7/1.6/1.6 | 17/5/3 |
| Ex. 2-26 | Ex. 1-10 | Activated carbon 0.1g | 100°C/3h | 7.7/7.1/7.8 | 1.5/1.4/1.6 | 18/4/3 |
| C. Ex. 2-1 | C. Ex. 1-1 | Activated carbon 0.1g | 100°C/3h | 100 | 6.1/6.4/6.2 | 121/19/38 |
| C. Ex. 2-2 | C. Ex. 1-2 | Activated carbon 0.1g | 100°C/3h | 100 | 7.2/7.3/7.0 | 115/16/33 |
| C. Ex. 2-3 | Ex. 1-1 | - | - | 100 | 6.3/6.8/6.9 | 106/21/43 |
| C. Ex. 2-4 | Ex. 1-2 | - | - | 100 | 6.6/6.9/7.0 | 119/18/39 |
| C. Ex. 2-5 | Ex. 1-3 | - | - | 100 | 7.1/7.3/7.5 | 109/18/35 |
| C. Ex. 2-6 | Ex. 1-4 | - | - | 100 | 7.3/7.2/7.4 | 105/20/41 |
| C. Ex. 2-7 | Ex. 1-5 | - | - | 100 | 7.8/7.7/7.1 | 111/16/34 |
| C. Ex. 2-8 | Ex. 1-6 | - | - | 100 | 8.0/8.3/8.3 | 103/17/38 |
| C. Ex. 2-9 | Ex. 1-7 | - | - | 100 | 8.2/8.4/8.0 | 107/16/32 |
| C. Ex. 2-10 | Ex. 1-8 | - | - | 100 | 7.1/7.3/7.5 | 100/14/31 |
| C. Ex. 2-11 | Ex. 1-9 | - | - | 100 | 7.9/8.0/8.1 | 92/10/24 |
| C. Ex. 2-12 | Ex. 1-10 | - | - | 100 | 8.3/8.3/8.6 | 117/15/33 |
| C. Ex. 2-13 | Ex. 1-11 | - | - | 100 | 7.6/7.8/8.0 | 125/14/30 |
| C. Ex. 2-14 | Ex. 1-12 | - | - | 100 | 7.2/7.3/7.6 | 121/17/37 |
| C. Ex. 2-15 | Ex. 1-13 | - | - | 100 | 7.7/8.0/8.1 | 120/16/41 |
| C. Ex. 2-16 | Ex. 1-14 | - | - | 100 | 7.9/7.8/8.2 | 131/11/33 |
| C. Ex. 2-17 | Ex. 1-15 | - | - | 100 | 8.0/7.7/8.0 | 112/10/30 |
| C. Ex. 2-18 | Ex. 1-16 | - | - | 100 | 7.6/8.0/7.3 | 108/13/29 |
| C. Ex. 2-19 | Ex. 1-17 | - | - | 100 | 7.3/7.5/7.5 | 125/11/33 |
| C. Ex. 2-20 | Ex. 1-18 | - | - | 100 | 7.7/7.6/8.0 | 116/10/39 |
| C. Ex. 2-21 | Ex. 1-19 | - | - | 100 | 7.3/7.3/7.6 | 111/16/40 |

As can be seen from Table 2 above, the purified and concentrated alcoholysis reaction compositions of Examples 2-1 to 2-26 had a very low pigment residual rate and a very low content of metals such as Sb, Ti, and Zn.

### Preparation of recycled terephthalic acid

### Example 3-1

A second high-pressure reactor with a capacity of 600 ml was charged with 100 g (0.36 mole) of the purified and concentrated alcoholysis reaction composition of Example 2-2 and 200 g (11.10 moles) of water, followed by an addition thereto of 50 mg of Zn(OAC)₂·2H₂O (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with butanol and water at 90°C and dried under vacuum to obtain 53.7 g (yield: 90%) of solid recycled terephthalic acid (TPA).

### Example 3-2

A second high-pressure reactor with a capacity of 600 ml was charged with 100 g (0.30 mole) of the purified and concentrated alcoholysis reaction composition of Example 2-5 and 200 g (11.10 moles) of water, followed by an addition thereto of 50 mg of Zn(OAC)₂·2H₂O (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with pentanol and water at 90°C and dried under vacuum to obtain 43.9 g (yield: 81%) of solid recycled terephthalic acid (TPA).

### Example 3-3

A second high-pressure reactor with a capacity of 600 ml was charged with 100 g (0.26 mole) of the purified and concentrated alcoholysis reaction composition of Example 2-11 and 1,000 g (55.50 moles) of water, followed by an addition thereto of 50 mg of Zn(OAC)₂·2H₂O (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with 2-ethyl-1-hexanol and water at 90°C and dried under vacuum to obtain 38.0 g (yield: 88%) of solid recycled terephthalic acid (TPA).

### Example 3-4

35.4 g (yield: 82%) of solid recycled terephthalic acid (TPA) was obtained in the same manner as in Example 3-3, except that 100 g (0.26 mole) of the purified and concentrated alcoholysis reaction composition of Example 2-13 was used.

### Example 3-5

36.7 g (yield: 85%) of solid recycled terephthalic acid (TPA) was obtained in the same manner as in Example 3-3, except that 100 g (0.26 mole) of the purified and concentrated alcoholysis reaction composition of Example 2-14 was used.

### Example 3-6

A second high-pressure reactor with a capacity of 600 ml was charged with 114 g (0.26 mole) of the purified and concentrated alcoholysis reaction composition of Example 2-18 and 200 g (11.10 moles) of water, followed by an addition thereto of 57 mg of Zn(OAC)₂·2H₂O (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with decanol and water at 90°C and dried under vacuum to obtain 36.1 g (yield: 85%) of solid recycled terephthalic acid (TPA).

### Example 3-7

54.3 g (yield: 91%) of solid recycled terephthalic acid (TPA) was obtained in the same manner as in Example 3-1, except that no hydrolysis catalyst was added.

### Example 3-8

A second high-pressure reactor with a capacity of 600 ml was charged with 100 g (0.36 mole) of the purified and concentrated alcoholysis reaction composition of Example 2-2 and 200 g (11.10 moles) of water, followed by an addition thereto of 50 mg of Zn(OAC)₂·2H₂O (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. Acetone was added to the solid obtained by filtering the hydrolysis reaction product in the form of a slurry, followed by stirring at 50°C for 4 hours. It was filtered, then washed with acetone and water at 50°C, and dried under vacuum to obtain 55.6 g (yield: 93%) of solid recycled terephthalic acid (TPA).

### Example 3-9

A second high-pressure reactor with a capacity of 600 ml was charged with 100 g (0.36 mole) of the purified and concentrated alcoholysis reaction composition of Example 2-2 and 200 g (11.10 moles) of water, followed by an addition thereto of 50 mg of Zn(OAC)₂·2H₂O (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. Butanol was added to the solid obtained by filtering the hydrolysis reaction product in the form of a slurry, followed by stirring at 90°C for 4 hours. It was filtered, then washed with butanol and water at 50°C, and dried under vacuum to obtain 53.1 g (yield: 89%) of solid recycled terephthalic acid (TPA).

### Example 3-10

59.2 g (yield: 99%) of solid recycled terephthalic acid (TPA) was obtained in the same manner as in Example 3-1, except that the filtrate separated through the filtration step in Example 3-1 was further used.

Specifically, the filtrate separated through the filtration step in Example 3-1 was charged to the second high-pressure reactor again, the temperature was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with butanol and water at 90°C and dried under vacuum to further obtain 5.5 g of solid recycled terephthalic acid (TPA).

### Comparative Example 3-1

54.4 g (yield: 91%) of solid recycled terephthalic acid (TPA) was obtained in the same manner as in Example 3-1, except that 100 g (0.36 mole) of the composition of Comparative Example 2-4 was used.

### Comparative Example 3-2

40.4 g (yield: 81%) of solid recycled terephthalic acid (TPA) was obtained in the same manner as in Example 3-2, except that 100 g (0.30 mole) of the composition of Comparative Example 2-7 was used.

### Comparative Example 3-3

36.7 g (yield: 85%) of solid recycled terephthalic acid (TPA) was obtained in the same manner as in Example 3-3, except that 100 g (0.26 mole) of the composition of Comparative Example 2-13 was used.

### Comparative Example 3-4

34.4 g (yield: 81%) of solid recycled terephthalic acid (TPA) was obtained in the same manner as in Example 3-6, except that 114 g (0.26 mole) of the composition of Comparative Example 2-16 was used.

### Test Example 2-1: Pigment residual rate

The terephthalic acid in each of Examples 3-1 to 3-10 and Comparative Examples 3-1 to 3-4 was measured for a pigment residual rate (%).

Specifically, the terephthalic acid of Example 3-1 was diluted to a concentration of 5% in a solvent, and the area (B1) of an absorbance curve was obtained in 400 nm to 800 nm using a UV-vis spectrophotometer. In such an event, the solvent used was dimethyl sulfoxide (DMSO), dimethyl formamide (DMF), or methyl pyrrolidone (NMP). In addition, for the composition of Comparative Example 2-4 (the composition of Example 1-2 used in the preparation of terephthalic acid of Example 3-1 was used whereas the composition was concentrated without adding an adsorbent), the area (B2) of an absorbance curve was obtained in 400 nm to 800 nm in the same manner as above. The pigment residual rate (%) was calculated according to the following Equation B using the areas of the measured absorbance curve. [Equation B] $Pigment residual rate of recycled terephthalic acid \left(%\right) = \frac{B 1}{B 2} \times 100$

The terephthalic acid in each of Examples 3-2 to 3-10 and Comparative Examples 3-1 to 3-4 was measured for a pigment residual rate (%) in the same manner as above. Specifically, as to B2 of Formula 1 above, Example 3-2 was measured using the composition of Comparative Example 2-7, Examples 3-3 to 3-5 were measured using the composition of Comparative Example 2-13, and Examples 3-6 was measured using the composition of Comparative Example 2-16. In addition, in Comparative Examples 3-1 to 3-4, B2 of Formula 1 was measured using the compositions of Comparative Examples 2-4, 2-7, 2-13, and 2-16, respectively.

### Test Example 2-2: Yellow index

The recycled terephthalic acid in each of Examples 3-1 to 3-10 and Comparative Examples 3-1 to 3-4 was measured for yellow index (Y.I.).

Specifically, the recycled terephthalic acid of each of Examples 3-1 to 3-10 and Comparative Examples 3-1 to 3-4 was diluted to a concentration of 5% in a solvent, and the yellow index was measured using ColorFlex EZ (manufacturer: HunterLab) equipment. In such an event, the solvent used was dimethyl sulfoxide (DMSO), dimethyl formamide (DMF), or methyl pyrrolidone (NMP).

### Test Example 2-3: Content of metals

The content (ppm) of metals present in the terephthalic acid in each of Examples 3-1 to 3-10 and Comparative Examples 3-1 to 3-10 was measured using inductively coupled plasma atomic emission spectroscopy (ICP-AES). Here, N.D. indicates that the content was too low, less than 1 ppm, to be measured as a specific value.

### Test Example 2-4: Color b

The terephthalic acid in each of Examples 3-1 to 3-10 and Comparative Examples 3-1 to 3-4 was measured for Color b, a color characteristic, using a spectrophotometer.

Specifically, the recycled terephthalic acid was measured in the reflectance mode with Illuminant D65 at an observer angle of 2° using a Varian Cary 5 UV/Vis/NIR spectrophotometer equipped with a diffuse reflectance accessory capable of measuring color and brightness to obtain data, and the Color b value was calculated by processing it with the color analysis device in the Grams/32 software.

**[Table 3]**

| | Composition | TPA yield (%) | Color b | Pigment residual rate (%) DMSO/DMF/NMP | Y.I. DMSO/DMF/ NMP | Metal content (ppm) (Sb/Ti/Zn) |
|---|---|---|---|---|---|---|
| Ex. 3-1 | Ex. 2-2 | 90 | 1.3 | 0/0/0 | 1.3/1.4/1.6 | N.D./N.D./N.D. |
| Ex. 3-2 | Ex. 2-5 | 81 | 1.0 | <0.5/<0.5/<0.5 | 1.1/0.9/1.0 | 1/N.D./N.D. |
| Ex. 3-3 | Ex. 2-11 | 88 | 1.3 | 0/0/0 | 1.1/1.2/1.3 | N.D./N.D./1 |
| Ex. 3-4 | Ex. 2-13 | 82 | 0.8 | 0/0/0 | 1.0/1.0/1.2 | 1/N.D./N.D. |
| Ex. 3-5 | Ex. 2-14 | 85 | 0.9 | 0/0/0 | 0.9/1.0/1.1 | N.D./N.D./N.D. |
| Ex. 3-6 | Ex. 2-18 | 85 | 1.4 | <0.5/<0.5/<0.5 | 1.2/1.2/1.1 | 2/N.D./N.D. |
| Ex. 3-7 | Ex. 2-2 | 91 | 1.2 | 0/0/0 | 1.1/1.1/1.3 | N.D./N.D./1 |
| Ex. 3-8 | Ex. 2-2 | 93 | 1.2 | 0/0/0 | 1.3/1.2/1.3 | 1/N.D./N.D. |
| Ex. 3-9 | Ex. 2-2 | 89 | 1.3 | 0/0/0 | 1.4/1.2/1.3 | N.D./N.D./N.D. |
| Ex. 3-10 | Ex. 2-2 | 99 | 1.4 | 0/0/0 | 1.6/1.5/1.7 | N.D./N.D./N.D. |
| C. Ex. 3-1 | C. Ex. 2-4 | 91 | 7.5 | 71/69/66 | 7.8/8.1/8.0 | 62/8/11 |
| C. Ex. 3-2 | C. Ex. 2-7 | 81 | 7.1 | 64/63/61 | 8.1/8.0/8.2 | 73/5/13 |
| C. Ex. 3-3 | C. Ex. 2-13 | 85 | 8.2 | 67/65/66 | 7.3/7.5/7.8 | 52/4/10 |
| C. Ex. 3-4 | C. Ex. 2-16 | 81 | 9.1 | 58/60/61 | 7.0/7.1/7.5 | 61/7/9 |

As can be seen from Table 3 above, the recycled terephthalic acid prepared in Examples 3-1 to 3-6 not only had a low pigment residual rate and a low yellow index, along with a Color b value satisfying a specific range, but also had a very low content of metals as impurities. In particular, in Example 3-10, the filtrate, that is, the unreacted material, separated through the filtration step was used to obtain recycled terephthalic acid in a very high yield.

### Preparation of a polyester resin

### Example 4-1

### (a) Preparation of a preliminary composition

A 10-liter reactor equipped with a column and a condenser that can be cooled by water was charged with the recycled terephthalic acid (r-TPA, 5,810.6 g) prepared in Example 3-1, recycled bis(2-hydroxyethyl) terephthalate (r-BHET, 3,810.4 g), ethylene glycol (EG, 1,333.1 g), cyclohexanedimethanol (CHDM, 864.1 g), isosorbide (ISB, 292.0 g), and diethylene glycol (DEG, 73.0 g). Here, G/A (total diol/total diacid; molar ratio of the glycol component to the diacid component) was 1.2.

Thereafter, 12.8 g of a Ge catalyst, 10.0 g of phosphoric acid as a stabilizer, 0.010 g of a blue toner, 0.005 g of a red toner, and 1,000 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 1.0 kgf/cm² (absolute pressure: 1,495.6 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 270°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.70 dl/g. The mixture was then discharged to the outside of the reactor to form strands, which were solidified with a cooling liquid and then granulated to have an average weight of about 12 mg to 14 mg.

### (d) Solid-state polymerization reaction

Subsequently, the granules of step (c) were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 200°C at a rate of 40°C/hour and maintained at 200°C. The solid-state polymerization reaction was carried out until the intrinsic viscosity (IV) of the granules in the reactor reached 1.30 dl/g to prepare a polyester resin.

### Example 4-2

### (a) Preparation of a preliminary composition

A 10-liter reactor equipped with a column and a condenser that can be cooled by water was charged with the recycled terephthalic acid (r-TPA, 8,419.6 g) prepared in Example 3-2, ethylene glycol (EG, 6,341.7 g), cyclohexanedimethanol (CHDM, 365.2 g), isosorbide (ISB, 98.7 g), and diethylene glycol (DEG, 148.1 g). Here, G/A (total diol/total diacid; molar ratio of the glycol component to the diacid component) was 2.1.

Thereafter, 6.4 g of a Ge catalyst, 0.030 g of a blue toner, and 0.015 g of a red toner were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 2.0 kgf/cm² (absolute pressure: 2,231.2 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 280°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.60 dl/g. The mixture was then discharged to the outside of the reactor to form strands, which were solidified with a cooling liquid and then granulated to have an average weight of about 12 mg to 14 mg.

### (d) Solid-state polymerization reaction

Subsequently, the granules of step (c) were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 210°C at a rate of 40°C/hour and maintained at 210°C. The solid-state polymerization reaction was carried out until the intrinsic viscosity (IV) of the granules in the reactor reached 0.85 dl/g to prepare a polyester resin.

### Example 4-3

### (a) Preparation of a preliminary composition

A 10-liter reactor equipped with a column and a condenser that can be cooled by water was charged with the recycled terephthalic acid (r-TPA, 4,195.3 g) prepared in Example 3-3, terephthalic acid (TPA, 4,195.3 g), ethylene glycol (EG, 4,794.8 g), cyclohexanedimethanol (CHDM, 145.6 g), and diethylene glycol (DEG, 369.0 g). Here, G/A (total diol/total diacid; molar ratio of the glycol component to the diacid component) was 1.6, and the molar ratio of r-TPA and TPA was 1:1.

Thereafter, 6.4 g of a Ge catalyst, 0.9 g of a Ti catalyst, 1.0 g of phosphoric acid as a stabilizer, 0.040 g of a blue toner, 0.010 g of a red toner, and 100 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 0.5 kgf/cm² (absolute pressure: 1,127.8 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 275°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.70 dl/g. The mixture was then discharged to the outside of the reactor to form strands, which were solidified with a cooling liquid and then granulated to have an average weight of about 12 mg to 14 mg to prepare a polyester resin.

### Example 4-4

### (a) Preparation of a preliminary composition

A 10-liter reactor equipped with a column and a condenser that can be cooled by water was charged with the recycled terephthalic acid (r-TPA, 2,486.5 g) prepared in Example 3-4, terephthalic acid (TPA, 4,973.0 g), recycled bis(2-hydroxyethyl) terephthalate (r-BHET, 1,268.2 g), ethylene glycol (EG, 2,600.4 g), cyclohexanedimethanol (CHDM, 431.4 g), and diethylene glycol (DEG, 364.5 g). Here, G/A (total diol/total diacid; molar ratio of the glycol component to the diacid component) was 1.15, and the molar ratio of r-TPA and TPA was 1:2.

Thereafter, 0.4 g of a Ti catalyst, 2.0 g of phosphoric acid as a stabilizer, 2.1 g of cobalt acetate as a colorant, and 200 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 1.0 kgf/cm² (absolute pressure: 1,495.6 mmHg). Then, the temperature of the reactor was raised from room temperature to 220°C over 90 minutes and maintained at 220°C for 2 hours, and the temperature was then raised again to 250°C over 2 hours. Thereafter, an esterification reaction was carried out at a temperature of 250°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 265°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.60 dl/g. The mixture was then discharged to the outside of the reactor to form strands, which were solidified with a cooling liquid and then granulated to have an average weight of about 12 mg to 14 mg.

### (d) Solid-state polymerization reaction

Subsequently, the granules of step (c) were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 220°C at a rate of 40°C/hour and maintained at 220°C. The solid-state polymerization reaction was carried out until the intrinsic viscosity (IV) of the granules in the reactor reached 1.00 dl/g to prepare a polyester resin.

### Example 4-5

### (a) Preparation of a preliminary composition

A 10-liter reactor equipped with a column and a condenser that can be cooled by water was charged with the recycled terephthalic acid (r-TPA, 754.8 g) prepared in Example 3-5, terephthalic acid (TPA, 1,887.0 g), recycled bis(2-hydroxyethyl) terephthalate (r-BHET, 7,507.3 g), ethylene glycol (EG, 1,268.6 g), cyclohexanedimethanol (CHDM, 2,095.3 g), and a compound comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3 (199.2 g). Here, G/A (total diol/total diacid; molar ratio of the glycol component to the diacid component) was 2.1, and the molar ratio of r-TPA and TPA was 1:2.5.

Thereafter, 12.8 g of a Ge catalyst, 0.9 g of a Ti catalyst, 1.0 g of phosphoric acid as a stabilizer, 1.3 g of cobalt acetate as a colorant; 0.006 g of a blue toner, 0.002 g of a red toner, and 100 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 2.0 kgf/cm² (absolute pressure: 2,231.2 mmHg). Then, the temperature of the reactor was raised from room temperature to 220°C over 90 minutes and maintained at 220°C for 2 hours, and the temperature was then raised again to 255°C over 2 hours. Thereafter, an esterification reaction was carried out at a temperature of 255°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 285°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.78 dl/g. The mixture was then discharged to the outside of the reactor to form strands, which were solidified with a cooling liquid and then granulated to have an average weight of about 12 mg to 14 mg to prepare a polyester resin.

### Example 4-6

### (a) Preparation of a preliminary composition

A 10-liter reactor equipped with a column and a condenser that can be cooled by water was charged with the recycled terephthalic acid (r-TPA, 6,640.6 g) prepared in Example 3-6, terephthalic acid (TPA, 368.9 g), recycled bis(2-hydroxyethyl) terephthalate (r-BHET, 564.5 g), ethylene glycol (EG, 3,279.4 g), cyclohexanedimethanol (CHDM, 1,984.2 g), and diethylene glycol (DEG, 648.9 g). Here, G/A (total diol/total diacid; molar ratio of the glycol component to the diacid component) was 1.7, and the molar ratio of r-TPA and TPA was 1:0.05.

Thereafter, 0.2 g of a Ti catalyst, 2.0 g of phosphoric acid as a stabilizer, 2.7 g of cobalt acetate as a colorant, and 200 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 1.5 kgf/cm² (absolute pressure: 1,863.4 mmHg). Then, the temperature of the reactor was raised from room temperature to 220°C over 90 minutes and maintained at 220°C for 2 hours, and the temperature was then raised again to 250°C over 2 hours. Thereafter, an esterification reaction was carried out at a temperature of 250°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 270°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.82 dl/g. The mixture was then discharged to the outside of the reactor to form strands, which were solidified with a cooling liquid and then granulated to have an average weight of about 12 mg to 14 mg to prepare a polyester resin.

### Example 4-7

### (a) Preparation of a preliminary composition

A 10-liter reactor equipped with a column and a condenser that can be cooled by water was charged with the recycled terephthalic acid (r-TPA, 5,958.1 g) prepared in Example 3-7, terephthalic acid (TPA, 1,051.4 g), ethylene glycol (EG, 898.9 g), cyclohexanedimethanol (CHDM, 4,256.4 g), isosorbide (ISB, 1,890.6 g), and diethylene glycol (DEG, 616.5 g). Here, G/A (total diol/total diacid; molar ratio of the glycol component to the diacid component) was 1.45, and the molar ratio of r-TPA and TPA was 1:0.18.

Thereafter, 64.0 g of a Ge catalyst, 0.2 g of phosphoric acid as a stabilizer, 0.030 g of a blue toner, 0.010 g of a red toner, and 20 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 1.0 kgf/cm² (absolute pressure: 1,495.6 mmHg). Then, the temperature of the reactor was raised from room temperature to 220°C over 90 minutes and maintained at 220°C for 2 hours, and the temperature was then raised again to 265°C over 2 hours. Thereafter, an esterification reaction was carried out at a temperature of 265°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 275°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.70 dl/g. The mixture was then discharged to the outside of the reactor to form strands, which were solidified with a cooling liquid and then granulated to have an average weight of about 12 mg to 14 mg to prepare a polyester resin.

### Example 4-8

### (a) Preparation of a preliminary composition

A 10-liter reactor equipped with a column and a condenser that can be cooled by water was charged with the recycled terephthalic acid (r-TPA, 1,545.8 g) prepared in Example 3-8, terephthalic acid (TPA, 3,864.4 g), recycled bis(2-hydroxyethyl) terephthalate (r-BHET, 3,547.8 g), ethylene glycol (EG, 721.7 g), cyclohexanedimethanol (CHDM, 536.4 g), diethylene glycol (DEG, 747.7 g), and a compound comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3 (407.9 g). Here, G/A (total diol/total diacid; molar ratio of the glycol component to the diacid component) was 1.1, and the molar ratio of r-TPA and TPA was 1:2.51.

Thereafter, 6.4 g of a Ge catalyst, 1.0 g of phosphoric acid as a stabilizer, 0.050 g of a blue toner, 0.020 g of a red toner, and 100 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 0.5 kgf/cm² (absolute pressure: 1,127.8 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 275°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.75 dl/g. The mixture was then discharged to the outside of the reactor to form strands, which were solidified with a cooling liquid and then granulated to have an average weight of about 12 mg to 14 mg to prepare a polyester resin.

### Example 4-9

### (a) Preparation of a preliminary composition

A 10-liter reactor equipped with a column and a condenser that can be cooled by water was charged with the recycled terephthalic acid (r-TPA, 3,788.8 g) prepared in Example 3-9, terephthalic acid (TPA, 421.0 g), isophthalic acid (IPA, 7,998.6 g), recycled bis(2-hydroxyethyl) terephthalate (r-BHET, 6,441.5 g), ethylene glycol (EG, 3,102.7 g), cyclohexanedimethanol (CHDM, 365.2 g), isosorbide (ISB, 98.7 g), and diethylene glycol (DEG, 370.3 g). Here, G/A (total diol/total diacid; molar ratio of the glycol component to the diacid component) was 2.1, and the molar ratio of r-TPA and TPA was 1:0.11.

Thereafter, 0.9 g of a Ti catalyst, 20.0 g of phosphoric acid as a stabilizer, 1.7 g of cobalt acetate as a colorant, and 2,000 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 3.0 kgf/cm² (absolute pressure: 2,966.8 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 280°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.60 dl/g. The mixture was then discharged to the outside of the reactor to form strands, which were solidified with a cooling liquid and then granulated to have an average weight of about 12 mg to 14 mg.

### (d) Solid-state polymerization reaction

Subsequently, the granules of step (c) were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 190°C at a rate of 40°C/hour and maintained at 190°C. The solid-state polymerization reaction was carried out until the intrinsic viscosity (IV) of the granules in the reactor reached 1.10 dl/g to prepare a polyester resin.

### Comparative Example 4-1

### (a) Preparation of a preliminary composition

A 10-liter reactor equipped with a column and a condenser that can be cooled by water was charged with the recycled terephthalic acid (r-TPA, 2,571.8 g) prepared in Comparative Example 3-1, terephthalic acid (TPA, 2,571.8 g), recycled bis(2-hydroxyethyl) terephthalate (r-BHET, 5,246.8 g), ethylene glycol (EG, 2,134.5 g), cyclohexanedimethanol (CHDM, 148.7 g), and isosorbide (ISB, 105.5 g). Here, G/A (total diol/total diacid; molar ratio of the glycol component to the diacid component) was 1.6, and the molar ratio of r-TPA and TPA was 1:1.

Thereafter, 12.8 g of a Ge catalyst was added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 0.5 kgf/cm² (absolute pressure: 1,127.8 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 280°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.50 dl/g. The mixture was then discharged to the outside of the reactor to form strands, which were solidified with a cooling liquid and then granulated to have an average weight of about 12 mg to 14 mg.

### (d) Solid-state polymerization reaction

Subsequently, the granules of step (c) were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 200°C at a rate of 40°C/hour and maintained at 200°C. The solid-state polymerization reaction was carried out until the intrinsic viscosity (IV) of the granules in the reactor reached 0.70 dl/g to prepare a polyester resin.

### Comparative Example 4-2

### (a) Preparation of a preliminary composition

A 10-liter reactor equipped with a column and a condenser that can be cooled by water was charged with the recycled terephthalic acid (r-TPA, 825.7 g) prepared in Comparative Example 3-2, terephthalic acid (TPA, 5,367.1 g), recycled bis(2-hydroxyethyl) terephthalate (r-BHET, 3,158.6 g), ethylene glycol (EG, 5,746.5 g), cyclohexanedimethanol (CHDM, 716.3 g), isosorbide (ISB, 193.7 g), and diethylene glycol (DEG, 72.6 g). Here, G/A (total diol/total diacid; molar ratio of the glycol component to the diacid component) was 2.5, and the molar ratio of r-TPA and TPA was 1:6.46.

Thereafter, 0.4 g of a Ti catalyst, 1.7 g of cobalt acetate as a colorant, and 10 ppm of a hindered phenol-based oxidation stabilizer (product name: Irganox 1076, manufacturer: BASF) were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 1.0 kgf/cm² (absolute pressure: 1,495.6 mmHg). Then, the temperature of the reactor was raised from room temperature to 220°C over 90 minutes and maintained at 220°C for 2 hours, and the temperature was then raised again to 260°C over 2 hours. Thereafter, an esterification reaction was carried out at a temperature of 260°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 280°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.70 dl/g. The mixture was then discharged to the outside of the reactor to form strands, which were solidified with a cooling liquid and then granulated to have an average weight of about 12 mg to 14 mg.

### (d) Solid-state polymerization reaction

Subsequently, the granules of step (c) were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 200°C at a rate of 40°C/hour and maintained at 200°C. The solid-state polymerization reaction was carried out until the intrinsic viscosity (IV) of the granules in the reactor reached 0.95 dl/g to prepare a polyester resin.

### Comparative Example 4-3

### (a) Preparation of a preliminary composition

A 10-liter reactor equipped with a column and a condenser that can be cooled by water was charged with the recycled terephthalic acid (r-TPA, 7,098.9 g) prepared in Comparative Example 3-3, ethylene glycol (EG, 2,854.7 g), cyclohexanedimethanol (CHDM, 3,079.0 g), isosorbide (ISB, 83.2 g), and diethylene glycol (DEG, 62.4 g). Here, G/A (total diol/total diacid; molar ratio of the glycol component to the diacid component) was 1.6.

Thereafter, 6.4 g of a Ge catalyst, 0.1 g of phosphoric acid as a stabilizer, 0.030 g of a blue toner, and 0.010 g of a red toner were added thereto, followed by stirring thereof to prepare a preliminary composition.

### (b) Esterification reaction

Subsequently, nitrogen was injected into the reactor containing the preliminary composition of step (a) to make the pressure in the reactor higher than normal pressure by 0.5 kgf/cm² (absolute pressure: 1,127.8 mmHg). Then, the temperature of the reactor was raised from room temperature to 220°C over 90 minutes and maintained at 220°C for 2 hours, and the temperature was then raised again to 255°C over 2 hours. Thereafter, an esterification reaction was carried out at a temperature of 255°C for 3 hours until the preliminary composition became transparent while the preliminary composition in the reactor was visually observed. Upon completion of the esterification reaction, nitrogen in the pressurized reactor was purged to the outside to lower the pressure in the reactor to normal pressure, and the product of the esterification reaction in the reactor was then transferred to a 7-liter reactor capable of a reaction under vacuum.

### (c) Polycondensation reaction

Thereafter, the pressure of the reactor containing the product of the esterification reaction of step (b) was reduced from normal pressure to 5.0 Torr (absolute pressure: 5 mmHg) over 30 minutes. At the same time, the temperature of the reactor was raised to 280°C over 1 hour, and a polycondensation reaction was carried out while the pressure of the reactor was maintained at 1.0 Torr (absolute pressure: 1.0 mmHg) or less. Here, the stirring speed was set high at the beginning of the polycondensation reaction. As the polycondensation reaction proceeded, when the stirring power was weakened due to the increase in the viscosity of the mixture in the reactor or the temperature of the mixture rose above the set temperature, the stirring speed was appropriately adjusted accordingly. The polycondensation reaction was carried out until the intrinsic viscosity (IV) of the mixture (melt) in the reactor reached 0.60 dl/g. The mixture was then discharged to the outside of the reactor to form strands, which were solidified with a cooling liquid and then granulated to have an average weight of about 12 mg to 14 mg to prepare a polyester resin.

### Test Example 3-1: Composition of the polyester resin

The polyester resins of Examples 4-1 to 4-9 and Comparative Examples 4-1 to 4-3 were each dissolved in a CDCl₃ solvent at a concentration of 3 mg/ml. The composition (% by mole) of the polyester resin was then measured through a 1H-NMR spectrum obtained using a nuclear magnetic resonance instrument (JEOL, 600 MHz FT-NMR) at 25°C.

### Test Example 3-2: Color characteristics

The polyester resins of Examples 4-1 to 4-9 and Comparative Examples 4-1 to 4-3 were each measured for Color b and Color L, which are color characteristics, using a spectrophotometer. Then, the Color L - Color b value, which is the difference between the Color L value and the Color b value, was calculated.

Specifically, the polyester resin was prepared into a specimen having a thickness of 6 mm. This specimen is measured in the transmission mode with Illuminant D65 at an observer angle of 2° using a Varian Cary 5 UV/Vis/NIR spectrophotometer equipped with a diffuse reflectance accessory capable of measuring color and brightness to obtain data, and the Hunter Color L, Color a, and Color b values are calculated by processing it with the color analysis device in the Grams/32 software. Then, the Color L - Color b value, which is the difference between the Color L value and the Color b value, was calculated.

**[Table 4]**

| | Content of r-TPA in the polyester resin (% by weight) | Molar ratio of r-TPA:TPA | Color L-b |
|---|---|---|---|
| Ex. 4-1 | 29 | r-TPA alone used | 93 |
| Ex. 4-2 | 42 | r-TPA alone used | 91 |
| Ex. 4-3 | 21 | 1:1 | 89 |
| Ex. 4-4 | 12 | 1:2 | 90 |
| Ex. 4-5 | 4 | 1:2.5 | 91 |
| Ex. 4-6 | 33 | 1:0.05 | 90 |
| Ex. 4-7 | 30 | 1:0.18 | 84 |
| Ex. 4-8 | 8 | 1:2.51 | 88 |
| Ex. 4-9 | 19 | 1:0.11 | 85 |
| C. Ex. 4-1 | 13 | 1:1 | 79 |
| C. Ex. 4-2 | 4 | 1:6.46 | 83 |
| C. Ex. 4-3 | 35 | r-TPA alone used | 81 |

As can be seen from Table 4 above, the polyester resins of Examples 4-1 to 4-9 had excellent color characteristics even though they each comprised recycled terephthalic acid (r-TPA) prepared from waste polyester. Specifically, as the polyester resins of Examples 4-1 to 4-9 each comprised the recycled terephthalic acid of Examples 3-1 to 3-9, which had excellent color characteristics by virtue of a low pigment residual rate and a low yellow index, along with a very low content of metal impurities, they had excellent quality. In particular, Example 2 showed excellent color characteristics despite the high content of recycled terephthalic acid in the polyester resin of 42% by weight without additional use of terephthalic acid.

## Claims

1. Recycled terephthalic acid, which is obtained by alcoholysis and hydrolysis of waste polyester and which has a Color b of less than 2 as measured in the reflection mode of a spectrophotometer.

2. The recycled terephthalic acid of claim 1, wherein the recycled terephthalic acid has a total content of metals of less than 100 ppm when measured by inductively coupled plasma atomic emission spectroscopy (ICP-AES).

3. The recycled terephthalic acid of claim 1, wherein the recycled terephthalic acid has a total content of Sb, Ti, and Zn of less than 30 ppm when measured by inductively coupled plasma atomic emission spectroscopy (ICP-AES).

4. The recycled terephthalic acid of claim 1, wherein the recycled terephthalic acid is obtained through a purification process and has a pigment residual rate of 10% or less according to the following Equation 1: [Equation 1] $Pigment residual rate of recycled terephthalic acid \left(%\right) = \frac{B 1}{B 2} \times 100$
in Equation 1, B1 is the area of an absorbance curve obtained in 400 nm to 800 nm using a UV-vis spectrophotometer for the recycled terephthalic acid as diluted to a concentration of 5% in dimethyl sulfoxide (DMSO), dimethyl formamide (DMF), or methylpyrrolidone (NMP), and
B2 is the area of an absorbance curve obtained in the same manner as above for recycled terephthalic acid that is obtained by the same preparation process as above but is not subjected to the purification process.

5. The recycled terephthalic acid of claim 1, wherein the yellow index (Y.I.) is less than 2 when the recycled terephthalic acid is diluted to a concentration of 5% in dimethyl sulfoxide (DMSO), dimethyl formamide (DMF), and methylpyrrolidone (NMP), respectively, and measured using a spectrophotometer.

6. A polyester resin, which comprises recycled terephthalic acid obtained by alcoholysis and hydrolysis of waste polyester and having a Color b of less than 2 as measured in the reflection mode of a spectrophotometer, and
which has a difference between the Color L value and the Color b value (Color L - Color b) of greater than 83 as measured in the transmission mode of a spectrophotometer for a specimen thereof having a thickness of 6 mm.

7. The polyester resin of claim 6, wherein the content of the recycled terephthalic acid is 0.1% by weight to 55% by weight based on the total weight of the polyester resin.

8. The polyester resin of claim 6, wherein the polyester resin comprises a diacid component and a glycol component,
the diacid component comprises at least one selected from the group consisting of terephthalic acid (TPA), isophthalic acid (IPA), dimethyl isophthalate, bis(2-hydroxyethyl) terephthalate, recycled bis(2-hydroxyethyl) terephthalate (r-BHET), phthalic acid, dimethyl terephthalate, phthalic anhydride, 2,6-naphthalenedicarboxylic acid, dimethyl 2,6-naphthalenedicarboxylate, diphenyl dicarboxylic acid, 1,4-cyclohexanedicarboxylic acid, 1,3-cyclohexanedicarboxylic acid, dimethyl 1,4-cyclohexanedicarboxylate, dimethyl 1,3-cyclohexanedicarboxylate, sebacic acid, succinic acid, isodecylsuccinic acid, maleic acid, maleic anhydride, fumaric acid, adipic acid, glutaric acid, and azelaic acid; and
the glycol compound comprises at least one selected from the group consisting of isosorbide (ISB), recycled isosorbide (r-ISB), ethylene glycol (EG), recycled ethylene glycol (r-EG), 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, 1,4-cyclohexanediol, cyclohexanedimethanol (CHDM) and its derivatives, recycled cyclohexanedimethanol (r-CHDM), diethylene glycol (DEG), recycled diethylene glycol (r-DEG), neopentyl glycol (NPG), and recycled neopentyl glycol (r-NPG).

9. The polyester resin of claim 8, wherein the molar ratio of the recycled terephthalic acid and the diacid component is 1:0.01 to 3.5.

10. The polyester resin of claim 6, wherein the polyester resin has an intrinsic viscosity (IV) of 0.5 dl/g to 1.3 dl/g.

11. An article, which comprises the polyester resin of claim 6.

12. A process for preparing a polyester resin, which comprises:
conducting alcoholysis and hydrolysis of waste polyester to prepare recycled terephthalic acid;
mixing the recycled terephthalic acid with a glycol component to prepare a preliminary composition;
subjecting the preliminary composition to an esterification reaction; and
subjecting the esterification reaction product to a polycondensation reaction,
wherein the recycled terephthalic acid has a Color b of less than 2 as measured in the reflection mode of a spectrophotometer.

13. The process for preparing a polyester resin of claim 12, wherein a diacid component is further mixed in the step of preparing the preliminary composition.

14. The process for preparing a polyester resin of claim 12, wherein the step of preparing the preliminary composition comprises:
(1) subjecting waste polyester to alcoholysis with an alcohol having 4 or more carbon atoms to prepare a liquid composition comprising a compound represented by the following Formula 1; and
(2) subjecting the liquid composition to hydrolysis: 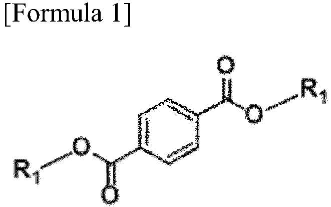 in Formula 1, R₁ is an alkyl group having 4 or more carbon atoms.

15. The process for preparing a polyester resin of claim 14, wherein the number of carbon atoms of the alcohol is 4 to 14.

16. The process for preparing a polyester resin of claim 14, which further comprises purifying the liquid composition before step (2).

17. The process for preparing a polyester resin of claim 16, wherein the purification step comprises adding at least one adsorbent selected from the group consisting of activated carbon, silica gel, alumina, zeolite, and activated clay, or adsorption through bed adsorption, and the content of the adsorbent added is 0.1% by weight to 20% by weight based on the total weight of the liquid composition.
